# EUROPÄISCHE PATENTANMELDUNG

(11) **EP 2 149 565 A1**
(43) Veröffentlichungstag der Anmeldung: **03.02.2010**
(21) Anmeldenummer: 08075664.6
(22) Anmeldetag: 24.07.2008
(51) Int. Cl.: C07D 239/94, A61K 31/517, A61P 37/02, A61P 37/08

(54) **Sulfonsubstituierte Chinazolinderivate als Immunmodulatoren zur Behandlung von enzündlichen und allergischen Erkrankungen**

(71) Anmelder: Bayer Schering Pharma Aktiengesellschaft, 13353 Berlin (DE)
(72) Erfinder: Nguyen, Duy, Dr., 10179 Berlin (DE); Buchmann, Bernd, Dr., 16540 Hohen Neuendorf (DE); Kosemund, Dirk, Dr., 07745 Jena (DE); Von Bonin, Arne, Dr., 16548 Glienicke-Nordbahn (DE)
(74) Vertreter: Bergmann, Martina

(57) **Zusammenfassung**

Die vorliegende Erfindung betrifft sulfonsubstituierte Chinazolinderivate der Formel (1), Verfahren zur deren Herstellung sowie deren Verwendung als Medikament zur Behandlung verschiedener Erkrankungen, die mit entzündlichen, allergischen und/oder proliferativen Prozessen einhergehen.

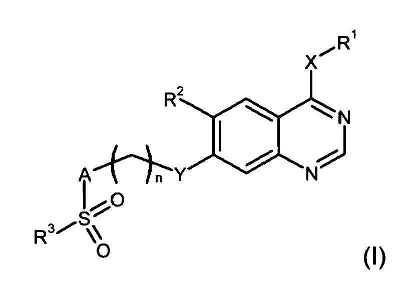

## Beschreibung

Die vorliegende Erfindung betrifft sulfonsubstituierte Chinazolinderivate, Verfahren zur deren Herstellung sowie deren Verwendung als Medikament zur Behandlung verschiedener Erkrankungen.

### Biologischer Hintergrund

Für zahlreiche chronisch entzündliche Erkrankungen, wie z.B. Rheumatoider Arthritis, Morbus Crohn, Asthma und Multipler Sklerose, ist ein überreagierendes Immunsystem mit verantwortlich. Durch ein vermehrtes Freisetzen von proinflammatorischen Zytokinen kommt es zu Schädigungen von körpereigenen Gewebestrukturen. Hierbei ist das Zusammenspiel von angeborenem und adaptiven Immunsystem von zentraler Bedeutung (Akira et al., 2001). Eine Modulation des Immunsystems mit Substanzen, die mit der Aktivierung von Zellen des angeborenen und/oder des adaptiven Immunsystem interferieren, wirkt anti-inflammatorisch und kann damit den pathologischen Phänotyp in den oben exemplarisch genannten Erkrankungen abmildern.
Die angeborene Immunität ("innate immunity") beruht darauf, daß Mikroorganismen wie Bakterien und Viren bestimmte inherente Merkmale aufweisen, durch die sie vom Immunsystem erkannt werden und anschließend aktivieren. Erkannt werden bestimmte Pathogen assozierte Muster ("pathogen associated molecular pattern, PAMPs"). PAMPs werden von den "Pattern recognition receptors" (PRR) erkannt, zu den auch Toll Like Rezeptoren (TLR) gehören (Janeway und Medzhitov, 2002). TLRs sind Homologe zum Drosophila-Rezeptorprotein "toll". Der Mensch hat zehn verschiedene TLRs. TLR eins und sechs sind Corezeptoren für TLR2. TLR2 erkennt u.a. Lipoproteine und Lipopeptide. TLR3 erkennt doppelsträngige RNA. TLR4 erkennt u.a. LPS von gram-negativen und Lipoteichonsäure von gram-positiven Bakterien. TLR5 erkennt Flagellin. TLR9 erkennt CpG-Motive in bakterieller DANN (O'Neill, 2006). Corezeptoren können die Erkennungsfähigkeiten von TLRs weiter verändern (Jiang et al., 2005).

### IL-1/-18, TLR Signaltransduktion

TLRs sind in der Signalübertragung mit IL-1 / IL-18 Zytokinrezeptoren verwandt. IL-1 ("endogenes Pyrogen") stimuliert stark Entzündung und induziert Fieber.
Mitglieder IL-1 R/TLR Superfamilie haben eine TIR Domäne (Toll/IL1 Receptor). Die TIR Domäne ist etwa 200 Aminosäuren lang und enthält drei konservierte Sequenzmotive. TIR-Domänen tragende Proteine binden über eine Protein-Protein Interaktion (O'Neill et al., 2005). Die Subklasse eins (IL-1R Familie) enthält drei Igartige-Domänen, der Rezeptor ist ein Heterodimer. Dazu gehören die IL-1 Rezeptoren eins und zwei, der Co-Rezeptor IL-1RAcP und die entsprechenden Proteine des IL-18 Systems. Die Subklasse zwei (TLR-Familie) enthält Leucin-rich Motife. Toll-like Rezeptoren bilden Homo- oder Heterodimere.

Nach Aktivierung der TLR bzw. IL-1, -18 Rezeptoren durch die entsprechenden Liganden wird eine mehrstufige Signalkaskade in Gang gesetzt. Der TLR bzw. IL-1/- 18 Rezeptorkomplex wechselwirkt über TIR/TIR Kontakte mit dem Adaptorprotein MyD88. Die "IL-1 associated receptor kinase" (IRAK-1) hat normalerweise Tollip (Toll interacting protein) gebunden, das wahrscheinlich als ein abschwächendes Molekül ("silencer") wirkt. IRAK/Tollip bindet an den aktiven TLR/IL-1 R Komplex. MyD88 verdrängt Tollip wodurch IRAK1 und IRAK-4 aktiviert wird, höchstwahrscheinlich als Dimer durch Transphosphorylierung. Aktives IRAK verlässt den Rezeptor und bindet im Cytoplasma an das Adaptermolekül TRAF (Barton und Medzhitov, 2003). Über TRAF werden weitere Proteine ubiquitiniliert. Über einen unbekannten Mechanismus führt Ub-TRAF zur Autophosphorylierung der S/T-Kinase TAK1 (eine MAP-Kinase Kinasekinase). TAK1 phosphoryliert IκB (NF-κB Aktivierung) und MKK6. Letztere ist für die Aktivierung der MAP-Kinasen p38 und JNK verantwortlich. NF-κB wurde als Nuclear Factor für die Expression der leichten Antikörperkette Kappa in B-Zellen identifiziert, ist aber an der Regulation vieler anderer Gene ebenfalls beteiligt. NF-κB wird im inaktiven Zustand im Cytoplasma zurückgehalten, wo es an den Inhibitor IκB gebunden ist (Deng et al., 2000). Phosphorylierung von IκB bewirkt, daß der Inhibitor IkB proteolytisch abgebaut wird und der Transkriptionsfaktor in den Kern wandern kann. NF-κB ist ein Heterodimer aus den Untereinheiten p65 (Rel) und p50 (Bäuerle und Henkel, 1994). Es gibt mehrere Mitglieder dieser Familie, die auf unterschiedliche Weise zusammenwirken können. NF-κB alleine kann Transkription nicht auslösen. Für Genaktivierung sind transkriptionelle Coaktivatoren erforderlich, wie etwa p300 oder CBT (Akira und Takeda, 2004).

### Den strukturell naheliegenden Stand der Technik bilden die Strukturen folgender Patentanmeldungen:

Benzyloxy-substituierte Chinazolinderivate werden in folgenden Patentanmeldungen genannt:. WO 2006/076246 (Inhibitoren von Serinproteasen), allerdings ist eine Amidseitenkette an der benzylischen Position zwingend notwendig. WO 93/17682 (Angiotensin-II-Rezeptorantagonisten). Die Sulfonylgruppe wird als Substituent nicht offenbart.
Ausgehend von diesem Stand der Technik besteht die Aufgabe der vorliegenden Erfindung, weitere Strukturen für die Therapie bereitzustellen, insbesondere für die Immunmodulation.

Die Aufgabe wird gelöst durch sulfonsubstituierte Verbindungen der allgemeinen Formel (I), 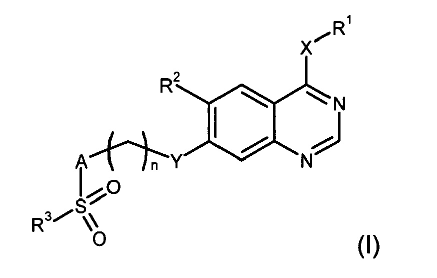 in der
- R¹: für
(i) einen gegebenenfalls mit Hydroxy, -NR⁶R⁷, -NR⁵-C(O)-R¹⁰, -NR⁵-C(O)-OR¹⁰, -NR⁵-C(O)-NR⁶R⁷, -NR⁶-SO₂-R¹⁰, Cyano, Halogen, C₁-C₆-Alkoxy, -OCF₃, -CF₃, C₁-C₆-Alkyl, C₃-C₆-Cycloalkyl und/oder Heterocyclyl mit 3 bis 8 Ringatomen ein- oder mehrfach, gleich oder verschieden substituierten Aryl- oder Heteroarylring, oder
(ii) einen gegebenenfalls mit Hydroxy, -NR⁶R⁷, -NR⁵-C(O)R¹⁰, -NR⁵-C(O)-OR¹⁰, -NR⁵-C(O)-NR⁶R⁷, -NR⁵-SO₂-R¹⁰, Cyano, Halogen, C₁-C₆-Alkoxy, -OCF₃, -CF₃, C₁-C₆-Alkyl, C₃-C₆-Cycloalkyl und/oder Heterocyclyl mit 3 bis 8 Ringatomen ein- oder mehrfach, gleich oder verschieden substituierten C₁-C₆-Alkyl-, C₂-C₆-Alkenyl- oder C₂-C₆-Alkinylrest, oder
(iii) einen gegebenenfalls mit Hydroxy, -NR⁶R⁷, -NR⁵-C(O)-R¹⁰, -NR⁵-C(O)-OR¹⁰, -NR⁵-C(O)-NR⁶R⁷, -NR⁵-SO₂-R¹⁰, Cyano, Halogen, C₁-C₆-Alkoxy, -OCF₃, -CF₃, C₁-C₆-Alkyl, C₃-C₆-Cycloalkyl und/oder Heterocyclyl mit 3 bis 8 Ringatomen ein- oder mehrfach, gleich oder verschieden substituierten C₃-C₈ Cycloalkyl- oder Heterocyclylring mit 3 bis 8 Ringatomen steht,
- R²: für
(i) Wasserstoff,
(ii) Hydroxy, Halogen, Cyano, Nitro, -CF₃, -OCF₃, -C(O)OR¹⁰, -C(O)OH, -C(O)NR⁶R⁷, -C(S)NR⁶R⁷, -NR⁶R⁷, -NR⁵-C(O)-R¹⁰, -NR⁵-C(O)-OR¹⁰, -NR⁵-C(O)-NR⁶R⁷, -NR⁵-SO₂-R¹⁰, oder
(iii) einen gegebenenfalls mit Halogen, Hydroxy, C₁-C₆-Alkoxy, -CF₃, -OCF₃ oder -NR⁶R⁷ ein- oder mehrfach, gleich oder verschieden substituierten C₁-C₆-Alkyl- oder C₁-C₆-Alkoxyrest, oder
(iv) einen gegebenenfalls mit Halogen, Hydroxy, C₁-C₆-Alkoxy, -CF₃, -OCF₃, -NR⁶R⁷ und/oder C₁-C₆-Alkyl ein- oder mehrfach, gleich oder verschieden substituierten C₃-C₈-Cycloalkylring steht,
- R³: für
einen C₁-C₆-Alkyl-, C₂-C₆-Alkenyl-, C₂-C₆-Alkinylrest, einen C₃-C₇-Cycloalkyl- oder Arylring, einen Heterocyclylring mit 3 bis 8 Ringatomen oder einen monocyclischen Heteroarylring steht, jeweils gegebenenfalls selbst mit Hydroxy, -C(O)OR¹⁰, -C(O)NR⁶R⁷, -NR⁶R⁷, Cyano, Halogen, -CF₃, C₁-C₆-Alkoxy, -OCF₃ und/oder C₁-C₆-Alkyl ein- oder mehrfach, gleich oder verschieden substituiert,
- X, Y: unabhängig voneinander für -O- oder die Gruppe -NR⁴- steht,
- A: für
(i) eine Bindung oder
(ii) einen gegebenenfalls mit Hydroxy, -NR⁶R⁷, -NR⁵-C(O)-R¹⁰, -C(O)NR⁶R⁷, -NR⁵-C(O)-OR¹⁰, -NR⁵-C(O)-NR⁶R⁷, -NR⁵-SO₂-R¹⁰, Cyano, Halogen, C₁-C₆-Alkoxy, -OCF₃, -CF₃, C₁-C₆-Alkyl, C₃-C₆-Cycloalkyl und/oder Heterocyclyl mit 3 bis 8 Ringatomen ein- oder mehrfach, gleich oder verschieden substituierten Aryl- oder Heteroarylring steht,
- n: für 1-6 steht,
- R⁴: für
(i) Wasserstoff,
(ii) einen C₁-C₆-Alkylrest, C₃-C₈-Cycloalkyl- oder Arylring, einen Heterocyclylring mit 3 bis 8 Ringatomen oder einen Heteroarylring, oder
(iii) -C(O)-(C₁-C₆)-Alkyl, -C(O)-Phenyl, oder -C(O)-Benzyl steht,
   wobei (ii) und (iii) gegebenenfalls mit Hydroxy, -NR⁸R⁹, Cyano, Halogen, -CF₃, C₁-C₆-Alkoxy und/oder -OCF₃ ein- oder mehrfach, gleich oder verschieden substituiert sind,
oder, wenn X für -NR⁴- steht, alternativ
X, R¹ und R⁴ gemeinsam einen 3 bis 8 gliedrigen Ring bilden, der gegebenenfalls zusätzlich zum Stickstoffatom ein oder mehrere weitere Heteroatome enthält, gegebenenfalls ein- oder mehrfach, gleich oder verschieden mit Hydroxy, C₁-C₆-Alkyl, C₁-C₆-Alkoxy, -C(O)R¹⁰, -SO₂R¹⁰, Halogen oder der Gruppe -NR⁸R⁹ substituiert ist, gegebenenfalls 1 bis 3 Doppelbindungen enthält und/oder gegebenenfalls durch eine oder mehrere -C(O)-Gruppen unterbrochen ist,
- R⁵: für Wasserstoff oder einen C₁-C₆-Alkylrest steht,
- R⁶ und R⁷: unabhängig voneinander stehen für
(i) Wasserstoff und/oder
(ii) einen C₁-C₆-Alkylrest, C₂-C₆-Alkenyl-, C₃-C₈-Cycloalkyl-und/oder Arylring, einen Heterocyclylring mit 3 bis 8 Ringatomen und/oder einen Heteroarylring, gegebenenfalls mit Hydroxy, -NR⁸R⁹, Cyano, Halogen, -CF₃, C₁-C₆-Alkoxy und/oder -OCF₃ ein- oder mehrfach, gleich oder verschieden substituiert sind, stehen oder
- R⁶ und R⁷: gemeinsam mit dem Stickstoffatom einen 5- bis 7-gliedrigen Ring bilden, der gegebenenfalls zusätzlich zum Stickstoffatom 1 oder 2 weitere Heteroatome enthält und der mit Hydroxy, -NR⁸R⁹, Cyano, Halogen, -CF₃, C₁-C₆-Alkyl, C₁-C₆-Alkoxy und/oder -OCF₃ ein- oder mehrfach, gleich oder verschieden substituiert sein kann,
- R⁸ und R⁹: unabhängig voneinander für Wasserstoff oder einen C₁-C₆-Alkylrest stehen, der gegebenenfalls mit Hydroxy oder Halogen ein- oder mehrfach, gleich oder verschieden substituiert ist,
- R¹⁰: für einen C₁-C₆-Alkyl, C₂-C₆-Alkenyl- oder C₂-C₆-Alkinylrest, einen C₃-C₈-Cycloalkyl- oder Arylring , einen Heterocyclylring mit 3 bis 8 Ringatomen oder einen Heteroarylring steht, jeweils gegebenenfalls selbst mit Hydroxy, Halogen, Cyano, Nitro, -NR⁶R⁷, C₁-C₆-Alkyl ,-CF₃, C₁-C₆-Alkoxy und/oder -OCF₃ ein- oder mehrfach, gleich oder verschieden substituiert,
sowie deren Salze, Diastereomere und Enantiomere.

Der Erfindung liegen folgende Definitionen zu Grunde:

### Cₙ-Alkyl:

Monovalenter, geradkettiger oder verzweigter, gesättigter Kohlenwasserstoffrest mit n Kohlenstoffatomen.

Ein C₁-C₆ Alkylrest umfasst unter anderem beispielsweise:
Methyl-, Ethyl-, Propyl-, Butyl-, Pentyl-,Hexyl-, iso-Propyl-, iso-Butyl-, sec-Butyl, tert-Butyl-, iso-Pentyl-, 2-Methylbutyl-, 1-Methylbutyl-, 1-Ethylpropyl-, 1,2-Dimethylpropyl, neo-Pentyl-, 1,1-Dimethylpropyl-, 4-Methylpentyl-, 3-Methylpentyl-, 2-Methylpentyl-, 1-Methylpentyl-, 2-Ethylbutyl-, 1-Ethylbutyl-, 3,3-Dimethylbutyl-, 2,2-Dimethylbutyl-, 1,1-Dimethylbutyl-, 2,3-Dimethylbutyl-, 1,3-Dimethylbutyl- 1,2-Dimethylbutyl-.

Bevorzugt ist ein Methyl-, Ethyl-, Propyl- oder Isopropylrest.

### Cₙ-Alkenyl:

monovalenter, geradkettiger oder verzweigter Kohlenwasserstoffrest mit n Kohlenstoffatomen und mindestens einer Doppelbindung.

### Ein C₂-C₆ Alkenylrest umfasst unter anderem beispielsweise:

Vinyl-, Allyl-, (E)-2-Methylvinyl-, (Z)-2-Methylvinyl-, Homoallyl-, (E)-But-2-enyl-, (Z)-But-2-enyl-, (E)-But-1-enyl-, (Z)-But-1-enyl-, Pent-4-enyl-, (E)-Pent-3-enyl-, (Z)-Pent-3-enyl-, (E)-Pent-2-enyl-, (Z)-Pent-2-enyl-, (E)-Pent-1-enyl-, (Z)-Pent-1-enyl-, Hex-5-enyl-, (E)-Hex-4-enyl-, (Z)-Hex-4-enyl-, (E)-Hex-3-enyl-, (Z)-Hex-3-enyl-, (E)-Hex-2-enyl-, (Z)-Hex-2-enyl-, (E)-Hex-1-enyl-, (Z)-Hex-1-enyl-, Isopropenyl-, 2-Methylprop-2-enyl-, 1-Methylprop-2-enyl-, 2-Methylprop-1-enyl-, (E)-1-Methylprop-1-enyl-, (Z)-1-Methylprop-1-enyl-, 3-Methylbut-3-enyl-, 2-Methylbut-3-enyl-, 1-Methylbut-3-enyl-, 3-Methylbut-2-enyl-, (E)-2-Methylbut-2-enyl-, (Z)-2-Methylbut-2-enyl-, (E)-1-Methylbut-2-enyl-, (Z)-1-Methylbut-2-enyl-, (E)-3-Methylbut-1-enyl-, (Z)-3-Methylbut-1-enyl-, (E)-2-Methylbut-1-enyl-, (Z)-2-Methylbut-1-enyl-, (E)-1-Methylbut-1-enyl-, (Z)-1-Methylbut-1-enyl-, 1,1-Dimethylprop-2-enyl-, 1-Ethylprop-1-enyl-, 1-Propylvinyl-, 1-Isopropylvinyl-, 4-Methylpent-4-enyl-, 3-Methylpent-4-enyl-, 2-Methylpent-4-enyl-, 1-Methylpent-4-enyl-, 4-Methylpent-3-enyl-, (E)-3-Methylpent-3-enyl-, (Z)-3-Methylpent-3-enyl-, (E)-2-Methylpent-3-enyl-, (Z)-2-Methylpent-3-enyl-, (E)-1-Methylpent-3-enyl-, (Z)-1-Methylpent-3-enyl-, (E)-4-Methylpent-2-enyl-, (Z)-4-Methylpent-2-enyl-, (E)-3-Methylpent-2-enyl-, (Z)-3-Methylpent-2-enyl-, (E)-2-Methylpent-2-enyl-, (Z)-2-Methylpent-2-enyl-, (E)-1-Methylpent-2-enyl-, (Z)-1-Methylpent-2-enyl-, (E)-4-Methylpent-1-enyl-, (Z)-4-Methylpent-1-enyl-, (E)-3-Methylpent-1-enyl-, (Z)-3-Methylpent-1-enyl-, (E)-2-Methylpent-1-enyl-, (Z)-2-Methylpent-1-enyl-, (E)-1-Methylpent-1-enyl-, (Z)-1-Methylpent-1-enyl-, 3-Ethylbut-3-enyl-, 2-Ethylbut-3-enyl-, 1-Ethylbut-3-enyl-, (E)-3-Ethylbut-2-enyl-, (Z)-3-Ethylbut-2-enyl-, (E)-2-Ethylbut-2-enyl-, (Z)-2-Ethylbut-2-enyl-, (E)-1-Ethylbut-2-enyl-, (Z)-1-Ethylbut-2-enyl-, (E)-3-Ethylbut-1-enyl-, (Z)-3-Ethylbut-1-enyl-, 2-Ethylbut-1-enyl-, (E)-1-Ethylbut-1-enyl-, (Z)-1-Ethylbut-1-enyl-, 2-Propylprop-2-enyl-, 1-Propylprop-2-enyl-, 2-Isopropylprop-2-enyl-, 1-Isopropylprop-2-enyl-, (E)-2-Propylprop-1-enyl-, (Z)-2-Propylprop-1-enyl-, (E)-1-Propylprop-1-enyl-, (Z)-1-Propylprop-1-enyl-, (E)-2-Isopropylprop-1-enyl-, (Z)-2-Isopropylprop-1-enyl-, (E)-1-Isopropylprop-1-enyl-, (Z)-1-Isopropylprop-1-enyl-, (E)-3,3-Dimethylprop-1-enyl-, (Z)-3,3-Dimethylprop-1-enyl-, 1-(1,1-Dimethylethyl)ethenyl.

Bevorzugt ist ein Vinyl- oder Allylrest.

### Cₙ-Alklnyl:

Monovalenter, geradkettiger oder verzweigter Kohlenwasserstoffrest mit n Kohlenstoffatomen und mindestens einer Dreifachbindung.

Ein C₂-C₆ Alkinylrest umfasst unter anderem beispielsweise:
Ethinyl-, Prop-1-inyl-, Prop-2-inyl-, But-1-inyl-, But-2-inyl-, But-3-inyl-, Pent-1-inyl-, Pent-2-inyl-, Pent-3-inyl-, Pent-4-inyl-, Hex-1-inyl-, Hex-2-inyl-, Hex-3-inyl-, Hex-4-inyl-, Hex-5-inyl-, 1-Methylprop-2-inyl-, 2-Methylbut-3-inyl-, 1-Methylbut-3-inyl-, 1-Methylbut-2-inyl-, 3-Methylbut-1-inyl-, 1-Ethylprop-2-inyl-, 3-Methylpent-4-inyl, 2-Methylpent-4-inyl, 1-Methylpent-4-inyl, 2-Methylpent-3-inyl, 1-Methylpent-3-inyl, 4-Methylpent-2-inyl, 1-Methylpent-2-inyl, 4-Methylpent-1-inyl, 3-Methylpent-1-inyl, 2-Ethylbut-3-inyl-, 1-Ethylbut-3-inyl-, 1-Ethylbut-2-inyl-, 1-Propylprop-2-inyl-, 1-Isopropylprop-2-inyl-, 2,2-Dimethylbut-3-inyl-, 1,1-Dimethylbut-3-inyl-, 1,1-Dimethylbut-2-inyl- oder eine 3,3-Dimethylbut-1-inyl-.

Bevorzugt ist ein Ethinyl-, Prop-1-inyl- oder Prop-2-inyl-rest.

### Cₙ-Cycloalkyl:

Monovalenter, cyclischer Kohlenwasserstoffring mit n Kohlenstoffatomen.

C₃-C₇-Cyclolalkylring umfasst:
Cyclopropyl, Cyclobutyl, Cyclopentyl, Cyclohexyl- und Cycloheptyl.

Bevorzugt ist ein Cyclopropyl-, Cyclobutyl-, Cylopentyl- oder ein Cyclohexylring.

### Cₙ-Alkoxy:

Geradkettiger oder verzweigter Cₙ-Alkyletherrest der Formel -OR mit R=Alkyl.

### Aryl

Aryl ist ein monovalentes, aromatisches Ringsystem ohne Heteroatom.
C₆-Aryl ist gleich Phenyl. C₁₀-Aryl ist gleich Naphthyl.
Wenn nicht anders angegeben umfasst Aryl nur Phenyl und Naphthyl.
Bevorzugt ist Phenyl.

### Heteroatome

Unter Heteroatomen sind Sauerstoff-, Stickstoff- oder Schwefel-Atome zu verstehen.

### Heteroaryl

Heteroaryl ist ein monovalentes, aromatisches Ringsystem mit mindestens einem von einem Kohlenstoff verschiedenen Heteroatom. Als Heteroatome können Stickstoffatome, Sauerstoffatome und/oder Schwefelatome vorkommen. Die Bindungsvalenz kann an einem beliebigen aromatischen Kohlenstoffatom oder an einem Stickstoffatom sein.

Wenn nicht anders angegeben umfasst Heteroaryl nur monocyclische und bicyclische Ringe.

Ein monocyclischer Heteroarylring gemäß der vorliegenden Erfindung hat 5 oder 6 Ringatome.

Heteroarylringe mit 5 Ringatomen umfassen beispielsweise die Ringe:
Thienyl, Thiazolyl, Furanyl, Pyrrolyl, Oxazolyl, Imidazolyl, Pyrazolyl, Isoxazolyl, Isothiazolyl, Oxadiazolyl, Triazolyl, Tetrazolyl- und Thiadiazolyl. Heteroarylringe mit 6 Ringatomen umfassen beispielsweise die Ringe: Pyridyl, Pyridazinyl, Pyrimidinyl, Pyrazinyl und Triazinyl. Ein bicyclischer Heteroarylring gemäß der vorliegenden Erfindung hat 9 oder 10 Ringatome.

Heteroarylringe mit 9 Ringatomen umfassen beispielsweise die Ringe:
Phthalidyl-, Thiophthalidyl-, Indolyl-, Isoindolyl-, Indazolyl-, Benzothiazolyl-, Indolonyl-, Isoindolonyl-, Benzofuranyl, Benzothienyl, Benzimidazolyl, Benzoxazolyl, Azocinyl, indolizinyl, Purinyl.

Heteroarylringe mit 10 Ringatomen umfassen beispielsweise die Ringe:
Isoquinolinyl-, Quinolinyl-, Benzoxazinonyl-, Phthalazinonyl, Quinolonyl-, Isoquinolonyl-, Quinazolinyl-, Quinoxalinyl-, Cinnolinyl-, Phthalazinyl-, 1,7- or 1,8-Naphthyridinyl-, Quinolinyl-, Isoquinolinyl-, Quinazolinyl- oder Quinoxalinyl-

Monocyclische Heteroarylringe mit 5 oder 6 Ringatomen sind bevorzugt.

### Heterocyclyl

Heterocyclyl im Sinne der Erfindung ist ein vollständig hydriertes Heteroaryl (vollständig hydriertes Heteroaryl = gesättigtes Heterocyclyl) d.h. ein nicht aromatisches Ringsystem mit mindestens einem von einem Kohlenstoff verschiedenen Heteroatom. Als Heteroatome können Stickstoffatome, Sauerstoffatome und/oder Schwefelatome vorkommen. Die Bindungsvalenz kann an einem beliebigen Kohlenstoffatom oder an einem Stickstoffatom sein. Heterocycylring mit 3 Ringatomen umfasst beispielsweise:
Aziridinyl.
Heterocycylring mit 4 Ringatomen umfasst beispielsweise:
   Azetidinyl, Oxetanyl.
   Heterocycylringe mit 5 Ringatomen umfassen beispielsweise die Ringe:
      Pyrrolidinyl, Imidazolidinyl , Pyrazolidinyl und Tetrahydrofuranyl.
      Heterocyclylringe mit 6 Ringatomen umfassen beispielsweise die Ringe:
         Piperidinyl, Piperazinyl, Morpholinyl, Tetrahydropyranyl und Thiomorpholinyl Heterocycylring mit 7 Ringatomen umfasst beispielsweise:
            Azepanyl, Oxepanyl, [1,3]-Diazepanly, [1,4]-Diazepanyl.
            Heterocycylring mit 8 Ringatomen umfasst beispielsweise:
               Oxocanyl, Azocanyl
               Wenn nicht anders angeben bedeutet Heterocyclyl ein Heterocyclylring mit 3 bis 8
               Ringatomen.

### Halogen

Die Bezeichnung Halogen umfasst Fluor, Chlor, Brom und lod.

Eine bevorzugte Untergruppe bilden Verbindungen der allgemeinen Formel (I), in der
- R¹: für
(i) einen gegebenenfalls mit Hydroxy, -NR⁶R⁷, Cyano, Halogen, C₁-C₆-Alkoxy, -NR⁵-C(O)R¹⁰, -OCF₃, -CF₃, C₁-C₆-Alkyl, ein- oder mehrfach, gleich oder verschieden substituierten Aryl- oder Heteroarylring, oder
(ii) einen gegebenenfalls mit Hydroxy, -NR⁶R⁷, Cyano, Halogen, C₁-C₆-Alkoxy, -NR⁵-C(O)R¹⁰, -OCF₃, -CF₃, C₁-C₆-Alkyl, ein- oder mehrfach, gleich oder verschieden substituierten C₁-C₆-Alkylrest, oder
(iii) einen gegebenenfalls mit Hydroxy, -NR⁶R⁷, Cyano, Halogen, C₁-C₆-Alkoxy, -OCF₃, -CF₃, C₁-C₆-Alkyl ein-oder mehrfach, gleich oder verschieden substituierten C₃-C₈ Cycloalkyl- oder Heterocyclylring mit 3 bis 8 Ringatomen steht,
- R²: für Wasserstoff, Halogen, Cyano, -C(O)OR¹⁰, -C(O)OH, -C(O)NR⁶R⁷ oder einen gegebenenfalls mit Halogen, Hydroxy, C₁-C₆-Alkoxy, -CF₃, -OCF₃ oder -NR⁶R⁷ ein- oder mehrfach, gleich oder verschieden substituierten C₁-C₆-Alkyl- oder C₁-C₆-Alkoxyrest steht,
- R³: für einen C₁-C₆-Alkylrest oder einen C₃-C₇-Cycloalkylring steht, gegebenenfalls selbst mit Hydroxy, -C(O)OR¹⁰, -C(O)NR⁶R⁷, -NR⁶R⁷, Cyano, Halogen, -CF₃, C₁-C₆-Alkoxy, -OCF₃ und/oder C₁-C₆-Alkyl ein- oder mehrfach, gleich oder verschieden substituiert,
- X: für die Gruppe -NR⁴- steht,
- Y: für -O- oder die Gruppe -NR⁴- steht,
- A: für
(i) eine Bindung oder
(ii) einen gegebenenfalls mit Hydroxy, -NR⁶R⁷, -C(O)NR⁶R⁷, Cyano, Halogen, C₁-C₆-Alkoxy, -OCF₃, -CF₃, C₁-C₆-Alkyl, C₃-C₆-Cycloalkyl und/oder Heterocyclyl mit 3 bis 8 Ringatomen ein- oder mehrfach, gleich oder verschieden substituierten Aryl- oder Heteroarylring steht,
- n: für 1-5 steht,
- R⁴: für
Wasserstoff, einen C₁-C₆-Alkylrest, einen C₃-C₈-Cycloalkylring oder -C(O)-(C₁-C₆)-Alkyl steht, jeweils gegebenenfalls mit Hydroxy, -NR⁸R⁹, Cyano, Halogen, -CF₃, C₁-C₆-Alkoxy und/oder -OCF₃ ein- oder mehrfach, gleich oder verschieden substituiert sind,
- R⁵: für Wasserstoff oder einen C₁-C₆-Alkylrest steht,
- R⁶ und R⁷: unabhängig voneinander stehen für
(i) Wasserstoff und/oder
(ii) einen C₁-C₆-Alkylrest, einen C₃-C₈-Cycloalkyl- und/oder Arylring, einen Heterocyclylring mit 3 bis 8 Ringatomen und/oder einen Heteroarylring,
   gegebenenfalls mit Hydroxy, -NR⁸R⁹, Cyano, Halogen, -CF₃, C₁-C₆-Alkoxy und/oder -OCF₃ ein- oder mehrfach, gleich oder verschieden substituiert sind,
- R⁸ und R⁹: unabhängig voneinander stehen für Wasserstoff oder einen C₁-C₃-Alkylrest,
- R¹⁰: für einen C₁-C₃-Alkyl, einen C₃-C₈-Cycloalkyl- oder Arylring , einen Heterocyclylring mit 3 bis 8 Ringatomen oder einen Heteroarylring steht,
jeweils gegebenenfalls selbst mit Hydroxy, Halogen, Cyano, Nitro, -NR⁶R⁷, C₁-C₆-Alkyl ,-CF₃, C₁-C₆-Alkoxy und/oder -OCF₃ ein- oder mehrfach, gleich oder verschieden substituiert,
sowie deren Salze, Diastereomere und Enantiomere.

Eine mehr bevorzugte Untergruppe bilden Verbindungen der allgemeinen Formel (I), in der
- R¹: für einen gegebenenfalls mit Hydroxy substituierten Aryl- oder Heteroarylring steht, oder
für einen gegebenenfalls mit -NR⁶R⁷ oder C₁-C₆-Alkoxy ein- oder mehrfach, gleich oder verschieden substituierten C₁-C₆-Alkylrest oder C₃-C₈ Cycloalkylring
- R²: für Wasserstoff, Halogen, -C(O)OR¹⁰, -C(O)OH oder einen C₁-C₆-Alkoxyrest steht,
- R³: für einen C₁-C₆-Alkylrest steht
- X: für -NH- steht,
- Y: für -O- steht,
- A: für eine Bindung oder einen Arylring steht,
- n: für 1-4 steht,
- R⁶ und R⁷: unabhängig voneinander stehen für Wasserstoff oder einen C₁-C₆-Alkylrest
- R¹⁰: für einen C₁-C₃-Alkylrest oder einen Arylring steht , jeweils gegebenenfalls selbst mit Nitro substituiert,
sowie deren Salze, Diastereomere und Enantiomere.

Eine ebenfalls mehr bevorzugte Untergruppe bilden die Verbindungen der allgemeinen Formel (I),
in der
- R¹: für
(i) einen gegebenenfalls mit Hydroxy, -NR⁵-C(O)-R¹⁰, Cyano, C₁-C₆-Alkyl ein- oder mehrfach, gleich oder verschieden substituierten Phenyl- oder monocyclischen Heteroarylring oder
(ii) einen gegebenenfalls mit Hydroxy, -NR⁶R⁷, C₁-C₆-Alkoxy, und/oder C₃-C₆-Cycloalkyl ein- oder mehrfach, gleich oder verschieden substituierten C₁-C₆-Alkylrest, oder
(iii) einen C₃-C₈ Cycloalkylring steht,
- R²: für Wasserstoff, Halogen, Cyano,-C(O)OR¹⁰, -C(O)OH, oder einen C₁-C₆-Alkoxyrest steht,
- R³: für einen C₁-C₆-Alkylrest steht,
- X: für -NH- steht,
- Y: für -O- oder -NH- steht,
- A: für eine Bindung oder einen Phenyl- oder monocyclischen Heteroarylring steht,
- n: für 1-4 steht,
- R⁵: für Wasserstoff steht,
- R⁶ und R⁷: unabhängig voneinander stehen für Wasserstoff oder einen C₁-C₆-Alkylrest, und
- R¹⁰: für einen C₁-C₆-Alkylrest oder Phenylring steht, jeweils gegebenenfalls selbst mit Nitro substituiert,
sowie deren Salze, Diastereomere und Enantiomere.

Eine besonders bevorzugte Untergruppe bilden die Verbindungen der allgemeinen Formel (I), in der
- R¹: für einen gegebenenfalls mit Hydroxy, -NR⁶R⁷, C₁-C₆-Alkoxy, und/oder C₃-C₆-Cycloalkyl ein- oder mehrfach, gleich oder verschieden substituierten C₁-C₆-Alkylrest,
- R²: für Wasserstoff, Halogen, Cyano oder einen C₁-C₆-Alkoxyrest steht,
- R³: für einen C₁-C₆-Alkylrest steht,
- X: für -NH- steht,
- Y: für -O- oder -NH- steht,
- A: für einen Phenyl- oder monocyclischen Heteroarylring steht,
- n: für 1 oder 2 steht, und
- R⁶ und R⁷: unabhängig voneinander stehen für Wasserstoff oder einen C₁-C₃-Alkylrest stehen,
sowie deren Salze, Diastereomere und Enantiomere.

Eine außerordentlich bevorzugte Untergruppe bilden die Verbindungen der allgemeinen Formel (I), in der
- R¹: für einen C₁-C₃-Alkylrest steht,
- R²: für Halogen oder einen C₁-C₆-Alkoxyrest steht,
- R³: für einen C₁-C₃-Alkylrest steht,
- X: für -NH- steht,
- Y: für -O- steht,
- A: für einen Phenylring steht, und
- n: für 1 steht,
sowie deren Salze, Diastereomere und Enantiomere.

In der allgemeinen Formel (I) kann R¹ stehen für
(i) einen gegebenenfalls mit Hydroxy, -NR⁶R⁷, -NR⁵-C(O)-R¹⁰, -NR⁵-C(O)-OR¹⁰, -NR⁵-C(O)-NR⁶R⁷, -NR⁶-SO₂-R¹⁰, Cyano, Halogen, C₁-C₆-Alkoxy, -OCF₃, -CF₃, C₁-C₆-Alkyl, C₃-C₆-Cycloalkyl und/oder Heterocyclyl mit 3 bis 8 Ringatomen ein- oder mehrfach, gleich oder verschieden substituierten Aryl- oder Heteroarylring, oder
(ii) einen gegebenenfalls mit Hydroxy, -NR⁶R⁷, -NR⁵-C(O)R¹⁰, -NR⁵-C(O)-OR¹⁰, -NR⁵-C(O)-NR⁶R⁷, -NR⁵-SO₂-R¹⁰, Cyano, Halogen, C₁-C₆-Alkoxy, -OCF₃, -CF₃, C₁-C₆-Alkyl, C₃-C₆-Cycloalkyl und/oder Heterocyclyl mit 3 bis 8 Ringatomen ein- oder mehrfach, gleich oder verschieden substituierten C₁-C₆-Alkyl-, C₂-C₆-Alkenyl- oder C₂-C₆-Alkinylrest, oder
(iii) einen gegebenenfalls mit Hydroxy, -NR⁶R⁷, -NR⁵-C(O)-R¹⁰, -NR⁵-C(O)-OR¹⁰, -NR⁵-C(O)-NR⁶R⁷, -NR⁵-SO₂-R¹⁰, Cyano, Halogen, C₁-C₆-Alkoxy, -OCF₃, -CF₃, C₁-C₆-Alkyl, C₃-C₆-Cycloalkyl und/oder Heterocyclyl mit 3 bis 8 Ringatomen ein- oder mehrfach, gleich oder verschieden substituierten C₃-C₈ Cycloalkyl- oder Heterocyclylring mit 3 bis 8 Ringatomen.

Bevorzugt steht R¹ für
(i) einen gegebenenfalls mit Hydroxy, -NR⁶R⁷, Cyano, Halogen, C₁-C₆-Alkoxy, -NR⁵-C(O)R¹⁰, -OCF₃, -CF₃, C₁-C₆-Alkyl, ein- oder mehrfach, gleich oder verschieden substituierten Aryl- oder Heteroarylring, oder
(ii) einen gegebenenfalls mit Hydroxy, -NR⁶R⁷, Cyano, Halogen, C₁-C₆-Alkoxy, -NR⁵-C(O)R¹⁰, -OCF₃, -CF₃, C₁-C₆-Alkyl, ein- oder mehrfach, gleich oder verschieden substituierten C₁-C₆-Alkylrest, oder
(iii) einen gegebenenfalls mit Hydroxy, -NR⁶R⁷, Cyano, Halogen, C₁-C₆-Alkoxy, -OCF₃, -CF₃, C₁-C₆-Alkyl ein- oder mehrfach, gleich oder verschieden substituierten C₃-C₈ Cycloalkyl- oder Heterocyclylring mit 3 bis 8 Ringatomen.

Mehr bevorzugt steht R¹ für :
(i) einen gegebenenfalls mit Hydroxy, -NR⁵-C(O)-R¹⁰, Cyano, C₁-C₆-Alkyl ein-oder mehrfach, gleich oder verschieden substituierten Phenyl- oder monocyclischen Heteroarylring oder
(ii) einen gegebenenfalls mit Hydroxy, -NR⁶R⁷, C₁-C₆-Alkoxy, und/oder C₃-C₆-Cycloalkyl ein- oder mehrfach, gleich oder verschieden substituierten C₁-C₆-Alkylrest, oder
(iii) einen C₃-C₈ Cycloalkylring.

Ebenso mehr bevorzugt steht R¹ auch für:
einen gegebenenfalls mit Hydroxy substituierten Aryl- oder Heteroarylring, oder für einen gegebenenfalls mit -NR⁶R⁷ oder C₁-C₆-Alkoxy ein- oder mehrfach, gleich oder verschieden substituierten C₁-C₆-Alkylrest oder C₃-C₈ Cycloalkylring.

Besonders bevorzugt steht R¹ für einen gegebenenfalls mit Hydroxy, -NR⁶R⁷, C₁-C₆-Alkoxy, und/oder C₃-C₆-Cycloalkyl ein- oder mehrfach, gleich oder verschieden substituierten C₁-C₆-Alkylrest, wobei R⁶ und R⁷ unabhängig voneinander für Wasserstoff oder einen C₁-C₃-Alkylrest stehen.

Außerordentlich bevorzugt steht R¹ für einen C₁-C₃-Alkylrest.

In der allgemeinen Formel (I) kann R² stehen für
(i) Wasserstoff,
(ii) Hydroxy, Halogen, Cyano, Nitro, -CF₃, -OCF₃, -C(O)OR¹⁰, -C(O)OH, -C(O)NR⁶R⁷, -C(S)NR⁶R⁷, -NR⁶R⁷, -NR⁵-C(O)-R¹⁰, -NR⁵-C(O)-OR¹⁰, -NR⁵-C(O)-NR⁶R⁷, -NR⁵-SO₂-R¹⁰, oder
(iii) einen gegebenenfalls mit Halogen, Hydroxy, C₁-C₆-Alkoxy, -CF₃, -OCF₃ oder -NR⁶R⁷ein- oder mehrfach, gleich oder verschieden substituierten C₁-C₆-Alkyl- oder C₁-C₆-Alkoxyrest, oder
(iv) einen gegebenenfalls mit Halogen, Hydroxy, C₁-C₆-Alkoxy, -CF₃, -OCF₃, -NR⁶R⁷und/oder C₁-C₆-Alkyl ein- oder mehrfach, gleich oder verschieden substituierten C₃-C₈-Cycloalkylring

Bevorzugt steht R² für
Wasserstoff, Halogen, Cyano, -C(O)OR¹⁰, -C(O)OH, -C(O)NR⁶R⁷oder einen gegebenenfalls mit Halogen, Hydroxy, C₁-C₆-Alkoxy, -CF₃, -OCF₃ oder -NR⁶R⁷ ein- oder mehrfach, gleich oder verschieden substituierten C₁-C₆-Alkyl- oder C₁-C₆-Alkoxyrest.

Mehr bevorzugt steht R² für:
Wasserstoff, Halogen, Cyano,-C(O)OR¹⁰, -C(O)OH oder einen C₁-C₆-Alkoxyrest.

Mehr bevorzugt steht R² auch für
Wasserstoff, Halogen, -C(O)OR¹⁰, -C(O)OH oder einen C₁-C₆-Alkoxyrest

Besonders bevorzugt steht R² für Wasserstoff, Halogen, Cyano oder einen C₁-C₆-Alkoxyrest.

Außerordentlich bevorzugt steht R² für Halogen oder einen C₁-C₆-Alkoxyrest.

In der allgemeinen Formel (I) kann R³ stehen für
einen C₁-C₆-Alkyl-, C₂-C₆-Alkenyl-, C₂-C₆-Alkinylrest, einen C₃-C₇-Cycloalkyl- oder Arylring, einen Heterocyclylring mit 3 bis 8 Ringatomen oder einen monocyclischen Heteroarylring, jeweils gegebenenfalls selbst mit Hydroxy, -C(O)OR¹⁰, -C(O)NR⁶R⁷, -NR⁶R⁷, Cyano, Halogen, -CF₃, C₁-C₆-Alkoxy, -OCF₃ und/oder C₁-C₆-Alkyl ein- oder mehrfach, gleich oder verschieden substituiert.

Bevorzugt steht R³ für
einen C₁-C₆-Alkylrest oder einen C₃-C₇-Cycloalkylring, gegebenenfalls selbst mit Hydroxy, -C(O)OR¹⁰, -C(O)NR⁶R⁷, -NR⁶R⁷, Cyano, Halogen, -CF₃, C₁-C₆-Alkoxy, -OCF₃ und/oder C₁-C₆-Alkyl ein- oder mehrfach, gleich oder verschieden substituiert.

Besonders bevorzugt steht R³ für einen C₁-C₆-Alkylrest.

Außerordentlich bevorzugt steht R³ für einen C₁-C₃-Alkylrest.

In der allgemeinen Formel (I) können X und Y unabhängig voneinander stehen für: -O- oder die Gruppe -NR⁴-.

Bevorzugt steht X für die Gruppe -NR⁴-.
Besonders bevorzugt steht X für -NH-.

Bevorzugt steht Y für -O- oder die Gruppe -NR⁴-.
Besonders bevorzugt steht Y für -O- oder -NH-.
Außerordentlich bevorzugt steht Y für -O-.

In der allgemeinen Formel (I) kann A stehen für
(i) eine Bindung oder
(ii) einen gegebenenfalls mit Hydroxy, -NR⁶R⁷, -NR⁵-C(O)-R¹⁰, -C(O)NR⁶R⁷, -NR⁵-C(O)-OR¹⁰, -NR⁵-C(O)-NR⁶R⁷, -NR⁵-SO₂-R¹⁰, Cyano, Halogen, C₁-C₆-Alkoxy, -OCF₃, -CF₃, C₁-C₆-Alkyl, C₃-C₆-Cycloalkyl und/oder Heterocyclyl mit 3 bis 8 Ringatomen ein- oder mehrfach, gleich oder verschieden substituierten Aryl- oder Heteroarylring .

Bevorzugt steht A für:
(i) eine Bindung oder
(ii) einen gegebenenfalls mit Hydroxy, -NR⁶R⁷, -C(O)NR⁶R⁷, Cyano, Halogen, C₁-C₆-Alkoxy, -OCF₃, -CF₃, C₁-C₆-Alkyl, C₃-C₆-Cycloalkyl und/oder Heterocyclyl mit 3 bis 8 Ringatomen ein- oder mehrfach, gleich oder verschieden substituierten Aryl- oder Heteroarylring.

Mehr bevorzugt steht A für :
für eine Bindung oder einen Phenyl- oder monocyclischen Heteroarylring.
Ebenfalls mehr bevorzugt steht A für eine Bindung oder einen Arylring.

Besonders bevorzugt steht A für einen Phenyl- oder monocyclischen Heteroarylring. Außerordentlich bevorzugt steht A für einen Phenylring.

In der allgemeinen Formel (I) kann n stehen für 1-6.
Bevorzugt steht n für 1-5.
Mehr bevorzugt steht n für 1-4.
Besonders bevorzugt steht n für 1 oder 2.
Außerordentlich bevorzugt steht n für 1.

In der allgemeinen Formel (I) kann R⁴ stehen für
(i) Wasserstoff,
(ii) einen C₁-C₆-Alkylrest, C₃-C₈-Cycloalkyl- oder Arylring, einen Heterocyclylring mit 3 bis 8 Ringatomen oder einen Heteroarylring, oder
(iii) -C(O)-(C₁-C₆)-Alkyl, -C(O)-Phenyl, oder -C(O)-Benzyl,
   wobei (ii) und (iii) gegebenenfalls mit Hydroxy, -NR⁸R⁹, Cyano, Halogen, -CF₃, C₁-C₆-Alkoxy und/oder -OCF₃ ein- oder mehrfach, gleich oder verschieden substituiert sind,
   oder, wenn X für -NR⁴- steht, alternativ
   X, R¹ und R⁴ gemeinsam einen 3 bis 8 gliedrigen Ring bilden, der gegebenenfalls zusätzlich zum Stickstoffatom ein oder mehrere weitere Heteroatome enthält, gegebenenfalls ein- oder mehrfach, gleich oder verschieden mit Hydroxy, C₁-C₆-Alkyl, C₁-C₆-Alkoxy, -C(O)R¹⁰, -SO₂R¹⁰, Halogen oder der Gruppe -NR⁸R⁹substituiert ist, gegebenenfalls 1 bis 3 Doppelbindungen enthält und/oder gegebenenfalls durch eine oder mehrere -C(O)-Gruppen unterbrochen ist,

Bevorzugt steht R⁴ für:
Wasserstoff, einen C₁-C₆-Alkylrest, einen C₃-C₈-Cycloalkylring oder -C(O)-(C₁-C₆)-Alkyl, jeweils gegebenenfalls mit Hydroxy, -NR⁸R⁹, Cyano, Halogen, -CF₃, C₁-C₆-Alkoxy und/oder -OCF₃ ein- oder mehrfach, gleich oder verschieden substituiert sind.

Besonders bevorzugt steht R⁴ für Wasserstoff.

In der allgemeinen Formel (I) kann R⁵ stehen für
Wasserstoff oder einen C₁-C₆-Alkylrest.
Bevorzugt steht R⁵ für Wasserstoff oder einen C₁-C₃-Alkylrest.
Besonders bevorzugt steht R⁵ für Wasserstoff.

In der allgemeinen Formel (I) können R⁶ und R⁷ unabhängig voneinander stehen für
(i) Wasserstoff und/oder
(ii) einen C₁-C₆-Alkylrest, C₂-C₆-Alkenyl-, C₃-C₈-Cycloalkyl- und/oder Arylring, einen Heterocyclylring mit 3 bis 8 Ringatomen und/oder einen Heteroarylring,
   gegebenenfalls mit Hydroxy, -NR⁸R⁹, Cyano, Halogen, -CF₃, C₁-C₆-Alkoxy und/oder -OCF₃ ein- oder mehrfach, gleich oder verschieden substituiert sind, oder

- R⁶und R⁷: bilden gemeinsam mit dem Stickstoffatom einen 5- bis 7-gliedrigen Ring, der gegebenenfalls zusätzlich zum Stickstoffatom 1 oder 2 weitere Heteroatome enthält und der mit Hydroxy, -NR⁸R⁹, Cyano, Halogen, -CF₃, C₁-C₆-Alkyl, C₁-C₆-Alkoxy und/oder -OCF₃ ein- oder mehrfach, gleich oder verschieden substituiert sein kann.

Bevorzugt stehen R⁶ und R⁷ unabhängig voneinander für:
(i) Wasserstoff und/oder
(ii) einen C₁-C₆-Alkylrest, einen C₃-C₈-Cycloalkyl- und/oder Arylring, einen Heterocyclylring mit 3 bis 8 Ringatomen und/oder einen Heteroarylring, gegebenenfalls mit Hydroxy, -NR⁸R⁹, Cyano, Halogen, -CF₃, C₁-C₆-Alkoxy und/oder -OCF₃ ein- oder mehrfach, gleich oder verschieden substituiert sind.

Besonders bevorzugt stehen R⁶ und R⁷ unabhängig voneinander für:
Wasserstoff oder einen C₁-C₆-Alkylrest.

Außerordentlich bevorzugt stehen R⁶ und R⁷ unabhängig voneinander stehen für:
Wasserstoff oder einen C₁-C₃-Alkylrest.

In der allgemeinen Formel (I) können R⁸ und R⁹ unabhängig voneinander stehen für:
Wasserstoff oder einen C₁-C₆-Alkylrest stehen, der gegebenenfalls mit Hydroxy oder Halogen ein- oder mehrfach, gleich oder verschieden substituiert ist.

Bevorzugt stehen R⁸ und R⁹ unabhängig voneinander für Wasserstoff oder einen C₁-C₃-Alkylrest.

In der allgemeinen Formel (I) kann R¹⁰ stehen für
für einen C₁-C₆-Alkyl, C₂-C₆-Alkenyl- oder C₂-C₆-Alkinylrest, einen C₃-C₈-Cycloalkyl-oder Arylring , einen Heterocyclylring mit 3 bis 8 Ringatomen oder einen Heteroarylring, jeweils gegebenenfalls selbst mit Hydroxy, Halogen, Cyano, Nitro, -NR⁶R⁷, C₁-C₆-Alkyl ,-CF₃, C₁-C₆-Alkoxy und/oder -OCF₃ ein- oder mehrfach, gleich oder verschieden substituiert.

Bevorzugt steht R¹⁰ für
für einen C₁-C₃-Alkyl, einen C₃-C₈-Cycloalkyl- oder Arylring , einen Heterocyclylring mit 3 bis 8 Ringatomen oder einen Heteroarylring.
jeweils gegebenenfalls selbst mit Hydroxy, Halogen, Cyano, Nitro, -NR⁶R⁷, C₁-C₆-Alkyl ,-CF₃, C₁-C₆-Alkoxy und/oder -OCF₃ ein- oder mehrfach, gleich oder verschieden substituiert.

Besonders bevorzugt steht R¹⁰ für:
einen C₁-C₆-Alkylrest oder einen Phenylring, jeweils gegebenenfalls selbst mit Nitro substituiert.

Ebenfalls besonders bevorzugt steht R¹⁰ für:
einen C₁-C₃-Alkylrest oder einen Arylring, jeweils gegebenenfalls selbst mit Nitro substituiert.

Ebenfalls als von der vorliegenden Erfindung als erfasst anzusehen sind alle Verbindungen, die sich ergeben durch jede mögliche Kombination der oben genannten möglichen, bevorzugten und besonders bevorzugten Bedeutungen der Substituenten.

Besondere Ausführungsformen der Erfindung bestehen darüber hinaus in Verbindungen, die sich durch Kombination der direkt in den Beispielen offenbarten Bedeutungen für die Substituenten ergeben.

Ebenfalls als von der vorliegenden Erfindung als erfasst anzusehen sind die Salze der Verbindungen.

Die Formulierung der erfindungsgemäßen Verbindungen zu pharmazeutischen Präparaten erfolgt in an sich bekannter Weise, indem man den oder die Wirkstoffe mit den in der Galenik gebräuchlichen Hilfsstoffen in die gewünschte Applikationsform überführt.

Als Hilfsstoffe können dabei beispielsweise Trägersubstanzen, Füllstoffe, Sprengmittel, Bindemittel, Feuchthaltemittel, Gleitmittel, Ab- und Adsorptionsmittel, Verdünnungsmittel, Lösungsmittel, Cosolventien, Emulgatoren, Lösungsvermittler, Geschmackskorrigentien, Färbemittel, Konservierungs-, Stabilisierungs-, Netzmittel, Salze zur Veränderung des osmotischen Drucks oder Puffer zum Einsatz kommen. Dabei ist auf Remington's Pharmaceutical Science, 15th ed. Mack Publishing Company, East Pennsylvania (1980) hinzuweisen.

Die pharmazeutischen Formulierungen können
in fester Form, zum Beispiel als Tabletten, Dragees, Pillen, Suppositorien, Kapseln, transdermale Systeme oder
in halbfester Form , zum Beispiel als Salben, Cremes, Gele, Suppositiorien, Emulsionen oder
in flüssiger Form, zum Beispiel als Lösungen, Tinkturen, Suspensionen oder Emulsionen vorliegen.

Hilfsstoffe im Sinne der Erfindung können beispielsweise Salze, Saccharide (Mono-, Di-, Tri-, Oligo-, und/oder Polysaccharide), Proteine, Aminosäuren, Peptide, Fette, Wachse, Öle, Kohlenwasserstoffe sowie deren Derivate sein, wobei die Hilfsstoffe natürlichen Ursprungs sein können oder synthetisch bzw. partial synthetisch gewonnen werden können.

Für die orale oder perorale Applikation kommen insbesondere Tabletten, Dragees, Kapseln, Pillen, Pulver, Granulate, Pastillen, Suspensionen, Emulsionen oder Lösungen in Frage.
Für die parenterale Applikation kommen insbesondere Suspensionen, Emulsionen und vor allem Lösungen in Frage.

Aufgrund ihrer anti-entzündlichen und zusätzlichen immunsuppressiven Wirkung können die erfindungsgemäßen Verbindungen der allgemeinen Formel (I) als Medikamente zur Behandlung oder Prophylaxe folgender Krankheitszustände bei Säugetieren und Menschen, für die lokale und systemische Applikation Verwendung finden:
(i) Lungenerkrankungen, die mit entzündlichen, allergischen und / oder proliferativen Prozessen einhergehen:
   - Chronisch obstruktive Lungenerkrankungen jeglicher Genese, vor allem Asthma bronchiale
   - Bronchitis unterschiedlicher Genese
   - Adult respiratory distress syndrome (ARDS), akutes Atemnotssyndrom
   - Bronchiektasen
   - Alle Formen der restriktiven Lungenerkrankungen, vor allem allergische Alveolitis,
   - Lungenödem, insbesondere allergisches
   - Sarkoidosen und Granulomatosen, insbesondere Morbus Boeck
(ii) Rheumatische Erkrankungen / Autoimmunerkrankungen / Gelenkerkrankungen, die mit entzündlichen, allergischen und / oder proliferativen Prozessen einhergehen:
   - Alle Formen rheumatischer Erkrankungen, insbesondere rheumatoide Arthritis, akutes rheumatisches Fieber, Polymyalgia rheumatica, Morbus Behcet
   - Reaktive Arthritis
   - Entzündliche Weichteilerkrankungen sonstiger Genese
   - Arthritische Symtome bei degenerativen Gelenkerkrankungen (Arthrosen)
   - Vitiligo
   - Kollagenosen jeglicher Genese, z.B. systemischer Lupus erythematodes, Sklerodermie, Polymyositis, Dermatomyositis- Sjögren-Syndrom, Still-Syndrom, Felty-Syndrom
   - Sarkoidosen und Granulomatosen
   - Weichteilrheumatismus
(iii) Allergien oder pseudoallergische Erkrankungen, die mit entzündlichen, und / oder proliferativen Prozessen einhergehen:
   - alle Formen allergischer Reaktionen, z.B. Quincke Ödem, Heuschnupfen, Insektenstich, allergische Reaktionen auf Arzneimittel, Blutderivate, Kontrastmittel etc., Anaphylaktischer Schock, Urtikaria, allergische und irritative Kontakdermatitis, allergische Gefäßerkrankungen
   - Vasculitis allergica
(iv) Gefäßentzündungen (Vaskulitiden)
   - Panarteriitis nodosa, Arteriitis temporalis, Erythema nodosum
   - Polyarteritis nodosa
   - Wegner Granulomatose
   - Riesenzellarteriitis
(v) Dermatologische Erkrankungen, die mit entzündlichen, allergischen und / oder proliferativen Prozessen einhergehen:
   - Atopische Dermatitis (vor allem bei Kindern)
   - Sämtliche Ekzemformen wie z.B. atopisches Ekzem (v.a. bei Kindern)
   - Exantheme jedweder Genese oder Dermatosen
   - Psoriasis und Parapsoriasis-Formenkreis
   - Pityriasis rubra pilaris
   - Erythematöse Erkrankungen, ausgelöst durch unterschiedlichen Noxen, z.B. Strahlen, Chemikalien, Verbrennungen etc.
   - Bullöse Dermatosen wie z.B. autoimmuner Pemphigus vulgaris, bullöses Pemphigoid
   - Erkrankungen des lichenoiden Formenkreises,
   - Pruritus (z. B. allergischer Genese)
   - Rosacea-Formenkreis
   - Erythema exsudativum multiforme
   - Manifestation von Gefäßerkrankungen
   - Haarausfall wie Alopecia areata
   - Kutane Lymphome
(vi) Nierenerkrankungen, die mit entzündlichen, allergischen und / oder proliferativen Prozessen einhergehen:
   - Nephrotisches Syndrom
   - Alle Nephritiden, z.B. Glomerulonephritis
(vii) Lebererkrankungen, die mit entzündlichen, allergischen und / oder proliferativen Prozessen einhergehen:
   - akute Hepatitis unterschiedlicher Genese
   - chronisch aggressive und / oder chronisch intermittierende Hepatitis
(viii) Gastrointestinale Erkrankungen, die mit entzündlichen, allergischen und / oder proliferativen Prozessen einhergehen:
   - regionale Enteritis (Morbus Crohn)
   - Colitis Ulcerosa
   - Gastroenteritiden anderer Genese, z.B. einheimische Sprue
(ix) Augenerkrankungen, die mit entzündlichen, allergischen und / oder proliferativen Prozessen einhergehen:
   - allergische Keratitis, Uveitis, Iritis,
   - Konjunktivitis
   - Blepharitis
   - Neuritis nervi optici
   - Chorioiditis
   - Ophthalmia sympathica
(x) Erkrankungen des Hals-Nasen-Ohren-Bereiches, die mit entzündlichen, allergischen und / oder proliferativen Prozessen einhergehen:
   - allergische Rhinitis, Heuschnupfen
   - Otitis externa, z.B. bedingt durch Kontaktekzem
(xi) Neurologische Erkrankungen, die mit entzündlichen, allergischen und / oder proliferativen Prozessen einhergehen:
   - Hirnödem, vor allem allergisches Hirnödem
   - Multiple Sklerose
   - akute Encephalomyelitis
   - Meningitis, vor allem allergische
   - Guillain-Barre Syndrom
   - Morbus Alzheimer
(xii) Bluterkrankungen, die mit entzündlichen, allergischen und / oder proliferativen Prozessen einhergehen wie z. B.: M. Hodgkin oder Non-Hodgkin Lymphome, Thrombozytaemien, Erythrozytosen
   - Erworbene hämolytische Anämie
   - Idiopathische Thrombozytopenie
   - Idiopathische Granulozytopenie
(xiii) Tumorerkrankungen, die mit entzündlichen, allergischen und / oder proliferativen Prozessen einhergehen
   - Akute lymphatische Leukämie
   - Maligne Lymphome
   - Lymphogranulomatosen
   - Lymphosarkome
(xiv) Endokrine Erkrankungen, die mit entzündlichen, allergischen und / oder proliferativen Prozessen einhergehen wie z. B.:
   - Endokrine Orbitopathie
   - Thyreoiditis de Quervain
   - Hashimoto Thyreoiditis
   - Morbus Basedow
   - Granulomatous thyroiditis
   - Struma lymphomatosa
   - Autoimmune adrenalitis
   - Diabetes mellitus, insbesondere Typ 1 Diabetes
   - Endometriose
(xv) Organ- und Gewebstransplantationen, Graft-versus-host-disease
(xvi) Schwere Schockzustände, z.B anaphylaktischer Schock, systemic inflammatory response syndrome (SIRS)

Ein Gegenstand der Erfindung ist die Verwendung der erfindungsgemäßen Verbindungen der allgemeinen Formel (I) zur Herstellung eines Arzneimittels.

Ein weiterer Gegenstand der Erfindung ist die Verwendung der erfindungsgemäßen Verbindungen zur Behandlung von Erkrankungen, die mit entzündlichen, allergischen und / oder proliferativen Prozessen einhergehen.

### Herstellung der erfindungsgemäßen Verbindungen

### Verfahrensvariante 1:

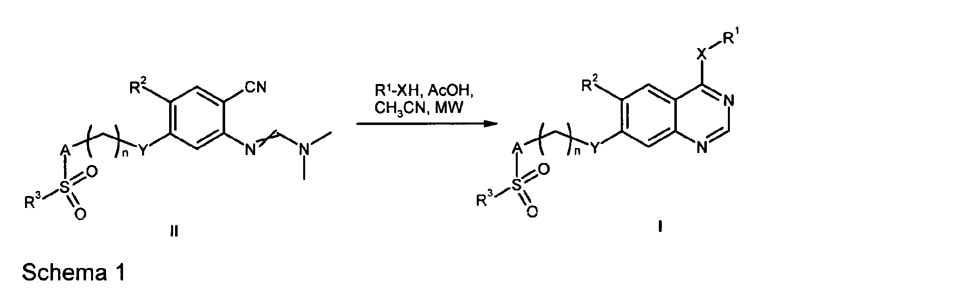

Gemäß Y. Hang et al.(Org. Lett., 2004, 6, 4775-4778) erfolgt die Darstellung der erfindungsgemäßen Verbindungen der allgemeinen Formel (I) durch Umsetzung der Intermediate gemäß Formel (II) mit Verbindungen R¹-XH in Gegenwart von Essigsäure in Acetonitril als Lösungsmittel in der Mikrowelle, wobei R¹, R², R³ sowie X, Y, A und n die in der allgemeinen Formel (I) gemäß der Ansprüche 1 bis 13 angegebenen Bedeutungen haben.

### Herstellung der Intermediate der Formel (II) :

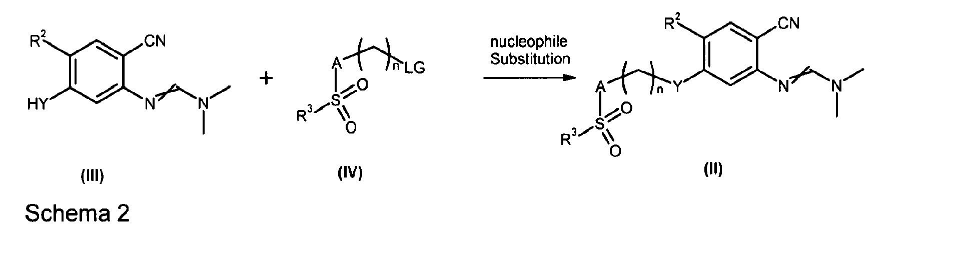

Die Substituenten R², R³ sowie Y, A und n haben die in der allgemeinen Formel (I) gemäß der Ansprüche 1 bis 13 angegebenen Bedeutungen.
Intermediate der Formel (II) erhält man durch eine nucleophile Substitutionsreaktion von Intermediaten der Formel (III) mit Intermediaten der Formel (IV). Intermediate der Formel (IV) sind dabei funktionalisiert mit einer für diesen Zweck geeigneten Gruppe LG. Geeignet als LG sind beispielsweise Halogen sowie eine Mesylat- ,Tosylat- oder Triflatgruppe. Für die Umsetzung der Intermediate (III) mit (IV) werden unter anderem Natrium-, Kalium- oder Cäsiumcarbonat als Base verwendet. Geeignete Lösungsmittel sind beispielsweise Aceton oder Dimethylformamid.

### Herstellung der Intermediate der Formel (III)

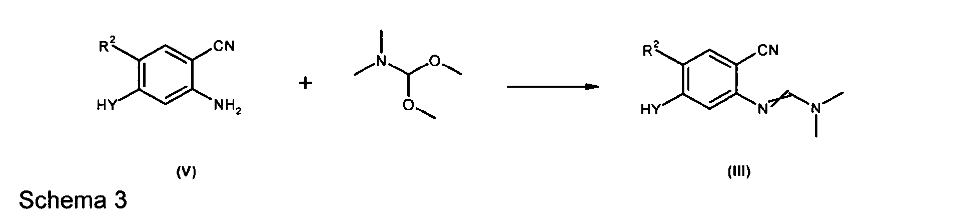

Intermediate der Formel (III) erhält man durch Umsetzung von Intermediaten der Formel (V) mit N,N-Dimethylformamiddimethylacetal, wobei R² und Y die in der allgemeinen Formel (I) gemäß der Ansprüche 1 bis 13 angegebenen Bedeutungen haben.

### Herstellung der Intermediate der Formel (IV)

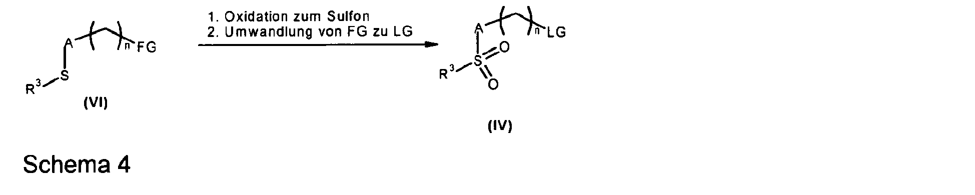

### 1. Oxidation zum Sulfon.

Ein Thioether der Formel (VI) wird zunächst in die entsprechende Sulfonverbindung überführt, wobei A und R³ die in der allgemeinen Formel (I) gemäß der Ansprüche 1 bis 13 angegebenen Bedeutungen haben. Geeignete Oxidationsmittel für diese Transformation sind beispielsweise Natriumperiodat, meta-Chlorperbenzoesäure, Wasserstoffperoxid oder Kaliumperoxomonosulfat. Die Oxidation zum Sulfon entfällt, wenn die entsprechende Sulfonverbindung bereits kommerziell erhältlich ist.

### 2. Umwandlung von FG zu LG

Funktionelle Gruppen FG sind beispielsweise Carbonsäure und Ester. Diese Gruppen können zum entsprechenden Alkohol reduziert werden. In einem nachfolgenden Schritt wird der Alkohol zu einer zur LG-Gruppe gehörenden Mesylat-, Tosylat und Triflatgruppe umgewandelt..

Im Falle für A = Aryl/Hetaryl und n =1 kann FG beispielsweise eine gegebenenfalls in geschützter Form vorliegenden Hydroxygruppe oder Wasserstoff bedeuten. Durch eine radikalische Halogenierung kann dieser Wasserstoff gegen einen Halogensubstituenten ausgetauscht werden.

### Verfahrensvariante 2:

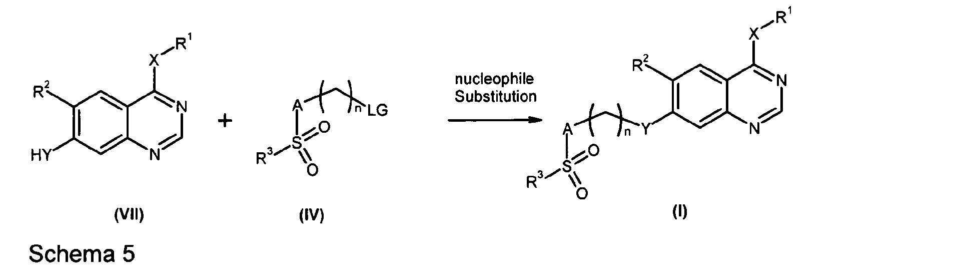

Die Darstellung der erfindungsgemäßen Verbindungen der allgemeinen Formel (I) erfolgt bei dieser Variante durch die Umsetzung der Chinazoline der Formel (VII) mit Intermediaten der Formel (IV), wobei R¹, R², R³ sowie X, Y, A und n die in der allgemeinen Formel (I) gemäß der Ansprüche 1 bis 13 angegebenen Bedeutungen haben. Die Reaktion erfolgt analog wie die Umsetzung der Intermediate der Formel (III) mit Intermediaten der Formel (IV) (vgl. Schema 2).

### Herstellung der Intermediate der Formel (VII)

Die Synthese der Chinazoline der Formel (VII) erfolgt analog wie in Verfahrensvariante 1 beschrieben (vgl. Schema 1) oder nach anderen dem Fachmann bekannten Methoden (vgl. hierzu Science of Synthesis, Houben-Weyl Methods of Molecular Transformations, Thieme Verlag, 2004, Band 16, Seite 573-749).

### Verfahrensvariante 3:

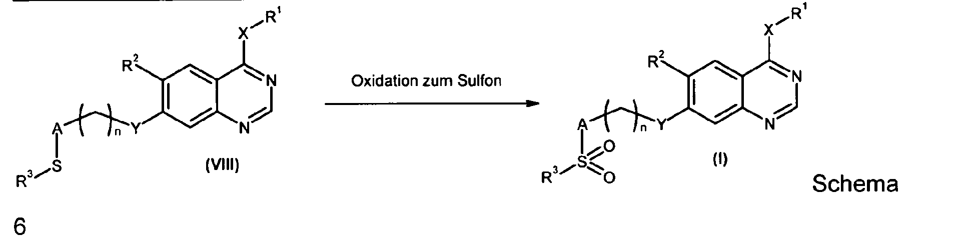

Bei dieser Verfahrensvariante können die erfindungsgemäßen Verbindungen der allgemeinen Formel (I) durch Oxidation am Schwefelzentrum der Verbindungen der Formel (VIII) zum entsprechenden Sulfon überführt werden, wobei R¹, R², R³,sowie X, Y, A und n die in der allgemeinen Formel (I) gemäß der Ansprüche 1 bis 13 angegebenen Bedeutungen haben.

### Herstellung der Intermediate der Formel (VIII) :

### Variante VIII-A

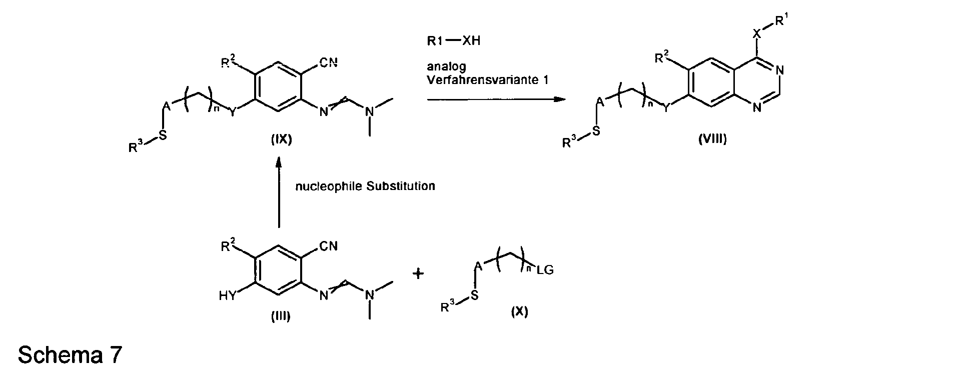

Intermediate der Formel (VIII) können analog Verfahrensvariante 1 hergestellt werden (vgl. Schema 1). Intermediate der Formel (IX) erhält man analog Schema 3 durch Umsetzung der Intermediate der Formel (III) mit Intermediaten der Formel (X). R¹, R², R³ sowie X, Y, A und n haben die in der allgemeinen Formel (I) gemäß der Ansprüche 1 bis 13 die angegebenen Bedeutungen.

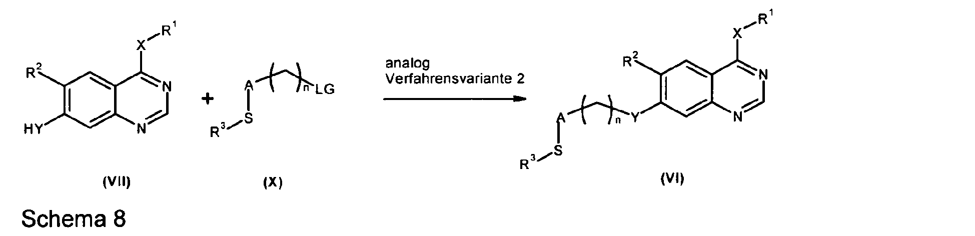

### Variante VIII-B

Alternativ und analog Verfahrensvariante 2 (vgl. Schema 5) lassen sich Intermediate der Formel (VIII) durch Reaktion der Intermediate der Formel (VII) mit Intermediaten der Formel (X) herstellen.
Geeignet als LG sind beispielsweise Halogen sowie eine Mesylat- ,Tosylat- oder Triflatgruppe sowie in diesem Fall auch eine Hydroxygruppe.
Ist LG eine Hydroxygruppe kann die Verknüpfung der Intermediate der Formel (V) mit Intermediaten der Formel (X) beispielsweise durch eine Mitsunobu-Reaktion erfolgen (O. Mitsunobu Synthesis 1981, 1-27).

### Experimenteller Teil:

### I. Synthese

### Verfahrensvariante 1

### Beispiel 1.1: [6-Brom-7-(3-methansulfonyl-benzyloxy)-chinazolin-4-yl]-isopropyl-amin

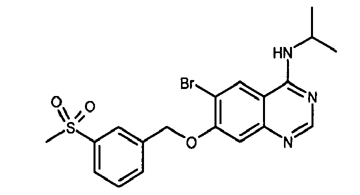

### 1.1 a) Herstellung der Zwischenprodukte

### Verbindung 1.1.a.1

### 2-Amino-5-brom-4-methoxybenzonitril

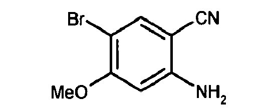

2-Amino-4-methoxybenzonitril (4.47 g, 30.2 mmol) wird in 70 mL Dioxan gelöst und bei 0°C mit Brom (1.71 mL, 33.2 mmol) versetzt. Es wird anschließend eine Stunde bei 0°C gerührt. Nach Zugabe von Diethylether werden die entstandenen Kristalle abgesaugt. Man erhält das gewünschte Produkt in 81 %iger Ausbeute (5.52 g).

¹H-NMR (400 MHz, DMSO-d6): δ 3.75 (s, 3H), 6.30-6.50 (m, 3H), 7.54 (s, 1 H).

### Verbindung 1.1.a.2

### (E/Z)-N'-(4-Brom-2-cyan-5-methoxyphenyl)-N, N-dimethylformimidamid

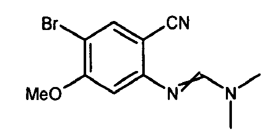

2-Amino-5-brom-4-methoxybenzonitril (3.0 g, 13.2 mmol) wird mit N,N-Dimethyl-formamiddimethylacetal (6.5 mL, 48.9 mmol) versetzt und anschließend 24 Stunden bei Raumtemperatur gerührt. Die Reaktionsmischung wird mehrmals mit Toluol zur Trockne eingeengt. Nach chromatographischer Reinigung (Kieselgel, Hexan/Ethylacetat: 0 →100% Ethylacetat, dann Ethylacetat/Methanol: 4/1) erhält man das gewünschte Produkt in 42%iger Ausbeute (1.56 g).

¹H-NMR (300 MHz, DMSO-d6): δ 2.95 (s, 3H), 3.05 (s, 3H), 3.87 (s, 3H), 6.80 (s, 1 H), 7.75 (s, 1 H), 7.99 (s, 1 H).

### Verbindung 1.1.a.3

### (E/Z)-N'-(4-Brom-2-cyan-5-hydroxyphenyl)-N,N-dimethylformimidamid

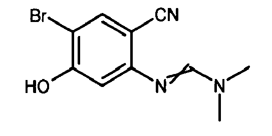

(E/Z)-N'-(4-Brom-2-cyan-5-methoxyphenyl)-N,N-dimethylformimidamid (1.28 g, 4.54 mmol) wird in 45 mL Methylenchlorid gelöst. Bortribromidlösung (1 M) in Methylenchlorid (91 mL, 91 mmol) wird hinzugetropft . Nach 20 Stunden bei Raumtemperatur wird die Reaktion durch Zugabe von Methanol zum Abbruch gebracht. Die Reaktionsmischung wird mehrmals mit Toluol zur Trockne eingeengt. Nach chromatographischer Reinigung (Kieselgel, Hexan/Ethylacetat: 0 **→**100% Ethylacetat, dann Ethylacetat/Methanol: 4/1) erhält man das gewünschte Produkt in 21 %iger Ausbeute (250 mg).

¹H-NMR (300 MHz, DMSO-d6): δ 2.92 (s, 3H), 3.02 (s, 3H), 6.50 (s, 1 H), 7.69 (s, 1 H), 7.75 (s, 1 H), 11.01 (br, 1 H).

### Verbindung 1.1.a.4

### (E/Z)-N'-[2-Cyano-5-(3-methansulfonyl-benzyloxy)-4-brom-phenyl]-N,N-dimethyl-formamidin

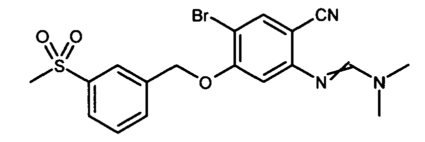

Zu einer Lösung von 1.0g 3-Methansulfonyltoluol in 5.9ml Tetrachlormethan wurde bei 25°C 1.05g N-Bromsuccinimid 11.8mg 2,2'-Azoisobutyronitril gegeben und anschließend 5 Stunden unter Rückfluß erhitzt. Es wurde abdekandiert und der orange Niederschlag mit Tetrachlormethan gewaschen und die vereinigten organischen Phasen im Vakuum eingeengt. Das so erhaltene 3-Brommethyl-phenylmethylsulfon wurde ohne weitere Reinigung in die nächste Stufe eingesetzt. 100mg (E/Z)-N'-(4-Brom-2-cyan-5-hydroxyphenyl)-N,N-dimethylformimidamid werden zusammen mit dem vorstehend frisch-hergestellten Bromids in 1.5ml Aceton suspendiert und nach Zugabe von 95.4mg Kaliumcarbonat für 3 Stunden unter Rückfluß erhitzt. Nach dem Abkühlen auf 25°C wurde mit 25ml Wasser versetzt und zweimal mit je 25ml Essigester gewaschen. Die vereinigten organischen Phasen wurden mit 25 ml gesättigter Natriumchlorid-Lösung gewaschen, über Natriumsulfat getrocknet und nach Filtration im Vakuum eingeengt. Der so erhaltene Rückstand wurde durch Säulenchromatographie an Kieselgel mit Essigester gereinigt. Es wurden 166mg (E/Z)-N'-[2-Cyano-5-(3-methansulfonyl-benzyloxy)-4-brom-phenyl]-N,N-dimethyl-formamidin erhalten

¹H-NMR (400 MHz, DMSO-d6): δ 2.38/2.67 (s, 3H), 2.96/3.07 (s, 3H), 3.17/3.21 (s, 3H), 4.61/5.36 (s, 2H), 6.96 (s) und 7.48 - 8.07 (m, 7H).

### 1.1 b) Herstellung des Endproduktes

166mg des unter 2a) hergestellten Zwischenproduktes wurden in 0.37ml Acetonitril gelöst. Nach Zugabe von 0.12ml Eisessig und 0.04ml Isopropylamin wurde für 20 Minuten bei 160°C in der Mikrowelle erhitzt. Die Reaktionsmischung wurde mit 40 ml verdünnter Natriumbicarbonat-Lösung versetzt und dreimal mit je 30 ml Essigester gewaschen. Die vereinigten organischen Phasen werden über Natriumsulfat getrocknet und nach Filtration im Vakuum eingeengt. Der so erhaltene Rückstand wurde durch Säulenchromatographie an Kieselgel mit Essigester / 0-1% Methanol gereinigt. Es wurden 79 mg [6-Brom-7-(3-methansulfonyl-benzyloxy)-chinazolin-4-yl]-isopropyl-amin erhalten.

¹H-NMR (400 MHz, DMSO-d6): δ 1.21 (d, 6H), 3.21 (s, 3H), 4.42 (dsept, 1H), 5.45 (d, 2H), 7.28 (s, 1 H), 7.70 (dd, 1 H), 7.84 (d, 1 H), 7.90 (m, 2H), 8.07 (s, 1 H), 8.38 (s, 1 H), 8.68 (s, 1 H).

### Verfahrensvariante 2

### Beispiel 2.1: Isopropyl-[7-(3-methansulfonyl-benzyloxy)-6-methoxy-chinazolin-4-yl]-amin

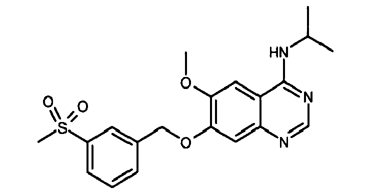

### 2.1 a) Herstellung des Zwischenproduktes

### 4-(Isopropylamino)-6-methoxychinazolin-7-ol

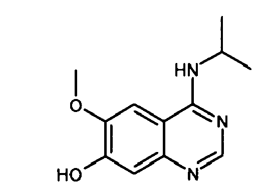

Das nach WO2004/58752 hergestellte (E/Z)-N'-(2-Cyan-5-hydroxy-4-methoxyphenyl)-N,N-dimethylformimidamid (0.62 g, 2.25 mmol) wird mit Isopropylamin (0.16 g, 2.7 mmol) in Acetonitril (3 mL) und Essigsäure (0.7 mL) versetzt und unter Rühren 10 Minuten bei 160°C mit Mikrowellen bestrahlt (vgl. Y. Hang, Org. Lett., 2004, 6, 4775-4778). Nach Abkühlen auf Raumtemperatur wird der Ansatz mittels gesättigter Natriumhydrogencatbonatlösung alkalisch gestellt und mit Ethylacetat extrahiert. Nach Einengen der organischen Phase erhält man das gewünschte Produkt in 55%iger Ausbeute (310 mg).

¹H-NMR (400 MHz, DMSO-d6): δ 1.26 (d, 6H), 3.91 (s, 3H), 4.49 (dsept, 1H), 6.94 (s, 1 H), 7.45 (d, 1 H), 7.59 (s, 1 H), 8.25 (s, 1 H), 10.08 (br s, 1 H).

### 2.1 b) Herstellung des Endproduktes

4-(Isopropylamino)-6-methoxychinazolin-7-ol (30 mg, 0.13 mmol) wird in 5 mL Aceton vorgelegt , mit nach US 2005/222175 und US2004-94-7995 hergestelltem 3-Methylsulfonylbenzylbromid (40 mg, 0.16 mmol) sowie Kaliumcarbonat (28 mg, 0.2 mmol) versetzt und anschließend 6 Stunden unter Rückfluss gekocht. Nach Abkühlen auf Raumtemperatur wir der Ansatz mit Ethylacetat verdünnt. Die organische Phase wird mit Wasser gewaschen und über Natriumsulfat getrocknet. Nach Einengen des Lösungsmittels sowie präparativer Dünnschichtchromatographie (Kieselgel, Dichlormethan/Methanol: 9/1) erhält man die gewünschte Verbindung in 40%iger Ausbeute (21 mg).

¹H-NMR (300 MHz, DMSO-d6): δ 1.28 (d, 6H), 3.25 s, 3H), 3.93 (s, 3H), 4.47-4.54 (m, 1 H), 5.38 (d, 2H), 7.21 (s, 1 H), 7.57 (d, 1 H), 7.67 (s, 1 H), 7.72 (t, 1 H), 7.85 (d, 1 H), 7.93 (d, 1 H), 8.08 (s, 1 H), 8.33 (s, 1 H).

### II. Biologische Wirkungen der erfindungsgemäßen Verbindungen

### TLR induzierte Zytokinfreisetzung in humanen "peripheral blood mononuclear cells" (PBMC)

### Versuchsprinzip

Aus humanem Vollblut isolierte PBMCs werden mit einem TLR Liganden stimuliert. Die Zytokin-Bestimmung wird mittels ELISA-Kits, eine Proliferations/Zellstoffwechselbestimmung wird mit Alamarblue durchgeführt.

### PBMC-Isolierung:

Für die Zellpräparation werden ca. 200 ml Blut mit einem Antikoagulanz (z.B. Citrat-Monovetten) versetzt. Pro Leucosep-Röhrchen werden 15ml Histopaque (Raumtemperatur, RT) eingefüllt und durch kurzes Anzentrifugieren (eine Minute bei 1000 x g, RT) durch die eingesetzte Fritte nach unten gedrückt. In die so vorbereiteten Röhrchen werden 20ml Blut gegeben und für 15 Minuten bei 800xg (RT) zentrifugiert. Nach der Zentrifugation ergibt sich von oben nach unten folgende Schichtung:
Plasma - PBMC - Histopaque - Filterscheibe - Histopaque - Erythrozyten und Granulozyten. Das überstehende Plasma wird abgesaugt. Die PBMC werden mitsamt dem darunterliegenden Histopaque in ein neues 50m1 Röhrchen überführt,
wobei immer der Inhalt von zwei Leucosep-Röhrchen
in ein 50ml Röhrchen gegeben wird. Die 50ml Röhrchen werden dann mit PBS auf 50ml aufgefüllt. Diese Zellsuspension wird für zehn Minuten bei 300xg (RT) zentrifugiert.
Der flüssige Überstand wird abgekippt und das Zellpellet mit wenig PBS resuspendiert und anschließend mit PBS auf 50ml aufgefüllt. Dieser Waschschritt wird zweimal wiederholt. Das entstandene Pellet wird in einem definierten Volumen von Medium (mit Zusätzen) aufgenommen. Zur Testung der Substanzen werden PBMC mit titrierten Konzentrationen der Testsubstanzen z.B. in Gegenwart oder Abwesenheit von TLR Liganden für 18 Stunden inkubiert. Am nächsten Tag werden die Überstände mittels spezifischer ELISA auf den Gehalt von IL-12, TNF-alpha oder anderen Chemokinen untersucht. Die Stoffwechselaktivität der behandelten Zellen wird mithilfe von Alamarblue bestimmt.

### Ergebnisse:

- **Beispiel**: **IC₅₀ (TNF-α)**
- **2.1**: **3 µM**

## Patentansprüche

1. Verbindungen der allgemeinen Formel (I) 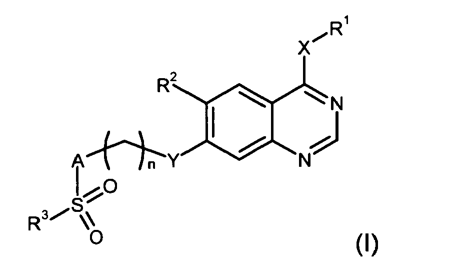 in der
R¹ für
(i) einen gegebenenfalls mit Hydroxy, -NR⁶R⁷, -NR⁵-C(O)-R¹⁰, -NR⁵-C(O)-OR¹⁰, -NR⁵-C(O)-NR⁶R⁷, -NR⁶-SO₂-R¹⁰, Cyano, Halogen, C₁-C₆-Alkoxy, -OCF₃, -CF₃, C₁-C₆-Alkyl, C₃-C₆-Cycloalkyl und/oder Heterocyclyl mit 3 bis 8 Ringatomen ein- oder mehrfach, gleich oder verschieden substituierten Aryl- oder Heteroarylring, oder
(ii) einen gegebenenfalls mit Hydroxy, -NR⁶R⁷, -NR⁵-C(O)R¹⁰, -NR⁵-C(O)-OR¹⁰, -NR⁵-C(O)-NR⁶R⁷, -NR⁵-SO₂-R¹⁰, Cyano, Halogen, C₁-C₆-Alkoxy, -OCF₃, -CF₃, C₁-C₆-Alkyl, C₃-C₆-Cycloalkyl und/oder Heterocyclyl mit 3 bis 8 Ringatomen ein- oder mehrfach, gleich oder verschieden substituierten C₁-C₆-Alkyl-, C₂-C₆-Alkenyl- oder C₂-C₆-Alkinylrest, oder
(iii) einen gegebenenfalls mit Hydroxy, -NR⁶R⁷, -NR⁵-C(O)-R¹⁰, -NR⁵-C(O)-OR¹⁰, -NR⁵-C(O)-NR⁶R⁷, -NR⁵-SO₂-R¹⁰, Cyano, Halogen, C₁-C₆-Alkoxy, -OCF₃, -CF₃, C₁-C₆-Alkyl, C₃-C₆-Cycloalkyl und/oder Heterocyclyl mit 3 bis 8 Ringatomen ein- oder mehrfach, gleich oder verschieden substituierten C₃-C₈ Cycloalkyl- oder Heterocyclylring mit 3 bis 8 Ringatomen steht,
R² für
(i) Wasserstoff,
(ii) Hydroxy, Halogen, Cyano, Nitro, -CF₃, -OCF₃, -C(O)OR¹⁰, -C(O)OH, -C(O)NR⁶R⁷, -C(S)NR⁶R⁷, -NR⁶R⁷, -NR⁵-C(O)-R¹⁰, -NR⁵-C(O)-OR¹⁰, -NR⁵-C(O)-NR⁶R⁷, -NR⁵-SO₂-R¹⁰, oder
(iii) einen gegebenenfalls mit Halogen, Hydroxy, C₁-C₆-Alkoxy, -CF₃, -OCF₃ oder -NR⁶R⁷ein- oder mehrfach, gleich oder verschieden substituierten C₁-C₆-Alkyl- oder C₁-C₆-Alkoxyrest, oder
(iv) einen gegebenenfalls mit Halogen, Hydroxy, C₁-C₆-Alkoxy, -CF₃,-OCF₃,-NR⁶R⁷ und/oder C₁-C₆-Alkyl ein- oder mehrfach, gleich oder verschieden substituierten C₃-C₈-Cycloalkylring
steht,
R³ für
einen C₁-C₆-Alkyl-, C₂-C₆-Alkenyl-, C₂-C₆-Alkinylrest, einen C₃-C₇-Cycloalkyl- oder Arylring, einen Heterocyclylring mit 3 bis 8 Ringatomen oder einen monocyclischen Heteroarylring steht, jeweils gegebenenfalls selbst mit Hydroxy, -C(O)OR¹⁰, -C(O)NR⁶R⁷, -NR⁶R⁷, Cyano, Halogen, -CF₃, C₁-C₆-Alkoxy, -OCF₃ und/oder C₁-C₆-Alkyl ein- oder mehrfach, gleich oder verschieden substituiert,
X, Y unabhängig voneinander für -O- oder die Gruppe -NR⁴- steht,
A für
(i) eine Bindung oder
(ii) einen gegebenenfalls mit Hydroxy, -NR⁶R⁷, -NR⁵-C(O)-R¹⁰, -C(O)NR⁶R⁷, -NR⁵-C(O)-OR¹⁰, -NR⁵-C(O)-NR⁶R⁷, -NR⁵-SO₂-R¹⁰, Cyano, Halogen, C₁-C₆-Alkoxy, -OCF₃, -CF₃, C₁-C₆-Alkyl, C₃-C₆-Cycloalkyl und/oder Heterocyclyl mit 3 bis 8 Ringatomen ein- oder mehrfach, gleich oder verschieden substituierten Aryl- oder Heteroarylring steht,
n für 1-6 steht,
R⁴ für
(i) Wasserstoff,
(ii) einen C₁-C₆-Alkylrest, C₃-C₈-Cycloalkyl- oder Arylring, einen Heterocyclylring mit 3 bis 8 Ringatomen oder einen Heteroarylring, oder
(iii) -C(O)-(C₁-C₆)-Alkyl, -C(O)-Phenyl, oder -C(O)-Benzyl steht,
wobei (ii) und (iii) gegebenenfalls mit Hydroxy, -NR⁸R⁹, Cyano, Halogen, -CF₃, C₁-C₆-Alkoxy und/oder -OCF₃ ein- oder mehrfach, gleich oder verschieden substituiert sind,
oder, wenn X für -NR⁴- steht, alternativ
X, R¹ und R⁴ gemeinsam einen 3 bis 8 gliedrigen Ring bilden, der gegebenenfalls zusätzlich zum Stickstoffatom ein oder mehrere weitere Heteroatome enthält, gegebenenfalls ein- oder mehrfach, gleich oder verschieden mit Hydroxy, C₁-C₆-Alkyl, C₁-C₆-Alkoxy, -C(O)R¹⁰, -SO₂R¹⁰, Halogen oder der Gruppe -NR⁷R⁸ substituiert ist, gegebenenfalls 1 bis 3 Doppelbindungen enthält und/oder gegebenenfalls durch eine oder mehrere -C(O)-Gruppen unterbrochen ist,
R⁵ für Wasserstoff oder einen C₁-C₆-Alkylrest steht,
R⁶ und R⁷ unabhängig voneinander stehen für
(i) Wasserstoff und/oder
(ii) einen C₁-C₆-Alkylrest, C₂-C₆-Alkenyl-, C₃-C₈-Cycloalkyl- und/oder Arylring, einen Heterocyclylring mit 3 bis 8 Ringatomen und/oder einen Heteroarylring, gegebenenfalls mit Hydroxy, -NR⁸R⁹, Cyano, Halogen, -CF₃, C₁-C₆-Alkoxy und/oder -OCF₃ ein- oder mehrfach, gleich oder verschieden substituiert sind, stehen oder
R⁶ und R⁷ gemeinsam mit dem Stickstoffatom einen 5- bis 7-gliedrigen Ring bilden, der gegebenenfalls zusätzlich zum Stickstoffatom 1 oder 2 weitere Heteroatome enthält und der mit Hydroxy, -NR⁸R⁹, Cyano, Halogen, -CF₃, C₁-C₆-Alkyl, C₁-C₆-Alkoxy und/oder -OCF₃ ein- oder mehrfach, gleich oder verschieden substituiert sein kann,
R⁸ und R⁹ unabhängig voneinander für Wasserstoff oder einen C₁-C₆-Alkylrest stehen, der gegebenenfalls mit Hydroxy oder Halogen ein- oder mehrfach, gleich oder verschieden substituiert ist,
R¹⁰ für einen C₁-C₆-Alkyl, C₂-C₆-Alkenyl- oder C₂-C₆-Alkinylrest, einen C₃-C₈-Cycloalkyl- oder Arylring , einen Heterocyclylring mit 3 bis 8 Ringatomen oder einen Heteroarylring steht, jeweils gegebenenfalls selbst mit Hydroxy, Halogen, Cyano, Nitro, -NR⁶R⁷, C₁-C₆-Alkyl ,-CF₃, C₁-C₆-Alkoxy und/oder -OCF₃ ein- oder mehrfach, gleich oder verschieden substituiert,
sowie deren Salze, Diastereomere und Enantiomere.

2. Verbindungen der allgemeinen Formel (I) gemäß Anspruch 1, in der
R¹ für
(i) einen gegebenenfalls mit Hydroxy, -NR⁶R⁷, Cyano, Halogen, C₁-C₆-Alkoxy, -NR⁵-C(O)R¹⁰, -OCF₃, -CF₃, C₁-C₆-Alkyl, ein- oder mehrfach, gleich oder verschieden substituierten Aryl- oder Heteroarylring, oder
(ii) einen gegebenenfalls mit Hydroxy, -NR⁶R⁷, Cyano, Halogen, C₁-C₆-Alkoxy, -NR⁵-C(O)R¹⁰, -OCF₃, -CF₃, Cₗ-C₆-Alkyl, ein- oder mehrfach, gleich oder verschieden substituierten C₁-C₆-Alkylrest, oder
(iii) einen gegebenenfalls mit Hydroxy, -NR⁶R⁷, Cyano, Halogen, C₁-C₆-Alkoxy, -OCF₃, -CF₃, C₁-C₆-Alkyl ein-oder mehrfach, gleich oder verschieden substituierten C₃-C₈ Cycloalkyl- oder Heterocyclylring mit 3 bis 8 Ringatomen steht,
R² für Wasserstoff, Halogen, Cyano, -C(O)OR¹⁰, -C(O)OH, -C(O)NR⁶R⁷ oder einen gegebenenfalls mit Halogen, Hydroxy, C₁-C₆-Alkoxy, -CF₃, -OCF₃ oder -NR⁶R⁷ ein- oder mehrfach, gleich oder verschieden substituierten C₁-C₆-Alkyl- oder C₁-C₆-Alkoxyrest steht,
R³ für einen C₁-C₆-Alkylrest oder einen C₃-C₇Cycloalkylring steht, gegebenenfalls selbst mit Hydroxy, -C(O)OR¹⁰, -C(O)NR⁶R⁷, -NR⁶R⁷, Cyano, Halogen, -CF_{3,} C₁-C₆-Alkoxy, -OCF₃ und/oder C₁-C₆-Alkyl ein- oder mehrfach, gleich oder verschieden substituiert,
X für die Gruppe -NR⁴- steht,
Y für -O- oder die Gruppe -NR⁴- steht,
A für
(i) eine Bindung oder
(ii) einen gegebenenfalls mit Hydroxy, -NR⁶R⁷, -C(O)NR⁶R⁷, Cyano, Halogen, C₁-C₆-Alkoxy, -OCF₃, -CF₃, C₁-C₆-Alkyl, C₃-C₆-Cycloalkyl und/oder Heterocyclyl mit 3 bis 8 Ringatomen ein- oder mehrfach, gleich oder verschieden substituierten Aryl- oder Heteroarylring steht,
n für 1-5 steht,
R⁴ für
Wasserstoff, einen C₁-C₆-Alkylrest, einen C₃-C₈-Cycloalkylring oder -C(O)-(C₁-C₆)-Alkyl steht, jeweils gegebenenfalls mit Hydroxy, -NR⁸R⁹, Cyano, Halogen, -CF₃, C₁-C₆-Alkoxy und/oder -OCF₃ ein- oder mehrfach, gleich oder verschieden substituiert sind,
R⁵ für Wasserstoff oder einen C₁-C₆-Alkylrest steht,
R⁶ und R⁷ unabhängig voneinander stehen für
(i) Wasserstoff und/oder
(ii) einen C₁-C₆-Alkylrest, einen C₃-C₈-Cycloalkyl- und/oder Arylring, einen Heterocyclylring mit 3 bis 8 Ringatomen und/oder einen Heteroarylring,
gegebenenfalls mit Hydroxy, -NR⁸R⁹, Cyano, Halogen, -CF₃, C₁-C₆-Alkoxy und/oder -OCF₃ ein- oder mehrfach, gleich oder verschieden substituiert sind,
R⁸ und R⁹ unabhängig voneinander stehen für Wasserstoff oder einen C₁-C₃-Alkylrest,
R¹⁰ für einen C₁-C₃-Alkyl, einen C₃-C₈-Cycloalkyl- oder Arylring , einen Heterocyclylring mit 3 bis 8 Ringatomen oder einen Heteroarylring steht,
jeweils gegebenenfalls selbst mit Hydroxy, Halogen, Cyano, Nitro, -NR⁶R⁷, C₁-C₆-Alkyl ,-CF₃, C₁-C₆-Alkoxy und/oder-OCF₃ ein- oder mehrfach, gleich oder verschieden substituiert,
sowie deren Salze, Diastereomere und Enantiomere.

3. Verbindungen der allgemeinen Formel (I) gemäß Anspruch 1 oder 2, in der
R¹ für einen gegebenenfalls mit Hydroxy, -NR⁶R⁷, C₁-C₆-Alkoxy, und/oder C₃-C₆-Cycloalkyl ein- oder mehrfach, gleich oder verschieden substituierten C₁-C₆-Alkylrest steht,
sowie deren Salze, Diastereomere und Enantiomere.

4. Verbindungen der allgemeinen Formel (I) gemäß einem der Ansprüche 1 bis 3, in der
R² für Halogen oder einen C₁-C₆-Alkoxyrest steht,
sowie deren Salze, Diastereomere und Enantiomere.

5. Verbindungen der allgemeinen Formel (I) gemäß einem der Ansprüche 1 bis 4, in der
R³ für einen C₁-C₃-Alkylrest steht,
sowie deren Salze, Diastereomere und Enantiomere.

6. Verbindungen der allgemeinen Formel (I) gemäß einem der Ansprüche 1 bis 5, in der
A für einen Phenylring steht,
sowie deren Salze, Diastereomere und Enantiomere.

7. Verbindungen der allgemeinen Formel (I) gemäß einem der Ansprüche 1 bis 6, in der
X für-NH- steht,
sowie deren Salze, Diastereomere und Enantiomere.

8. Verbindungen der allgemeinen Formel (I) gemäß einem der Ansprüche 1 bis 7, in der
Y für -O- steht,
sowie deren Salze, Diastereomere und Enantiomere.

9. Verbindungen der allgemeinen Formel (I) gemäß einem der Ansprüche 1 bis 8, in der
n für 1 steht,
sowie deren Salze, Diastereomere und Enantiomere.

10. Verbindungen gemäß Anspruch 1 der allgemeinen Formel (I), in der
R¹ für einen gegebenenfalls mit Hydroxy, -NR⁶R⁷, C₁-C₆-Alkoxy, und/oder C₃-C₆-Cycloalkyl ein- oder mehrfach, gleich oder verschieden substituierten C₁-C₆-Alkylrest,
R² für Wasserstoff, Halogen, Cyano oder einen C₁-C₆-Alkoxyrest steht,
R³ für einen C₁-C₆-Alkylrest steht,
X für -NH- steht,
Y für -O- oder -NH- steht,
A für einen Phenyl- oder monocyclischen Heteroarylring steht,
n für 1 oder 2 steht, und
R⁶ und R⁷ unabhängig voneinader für Wasserstroff oder einen C₁-C₃-Alkylrest stehen,
sowie deren Salze, Diastereomere und Enantiomere.

11. Verbindungen gemäß Anspruch 1 der allgemeinen Formel (I), in der
R¹ für einen C₁-C₃-Alkylrest steht,
R² für Halogen oder einen C₁-C₆-Alkoxyrest steht,
R³ für einen C₁-C₃-Alkylrest steht,
X für -NH- steht,
Y für -O- steht,
A für einen Phenylring steht, und
n für 1 steht,
sowie deren Salze, Diastereomere und Enantiomere.

12. Verfahren zur Herstellung der Verbindungen gemäß einem der Ansprüche 1 bis 11, umfassend den Schritt, der Intermediate gemäß Formel (II) mit Verbindungen R¹-XH umsetzt, 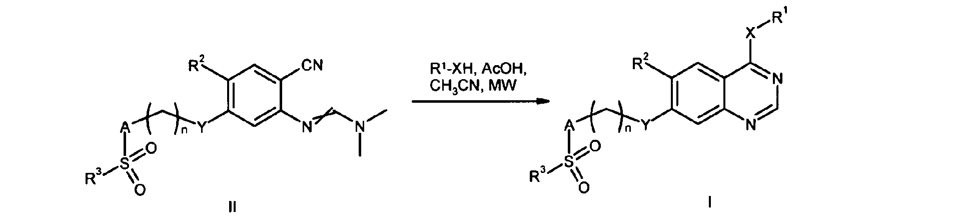 wobei R¹, R², R³ sowie X, Y, A und n die in der allgemeinen Formel (I) gemäß der Ansprüche 1 bis 11 angegebenen Bedeutungen haben.

13. Verfahren zur Herstellung der Verbindungen gemäß einem der Ansprüche 1 bis 11, umfassend den Schritt, der Chinazoline der Formel (VII) mit Intermediaten der Formel (IV) umsetzt, 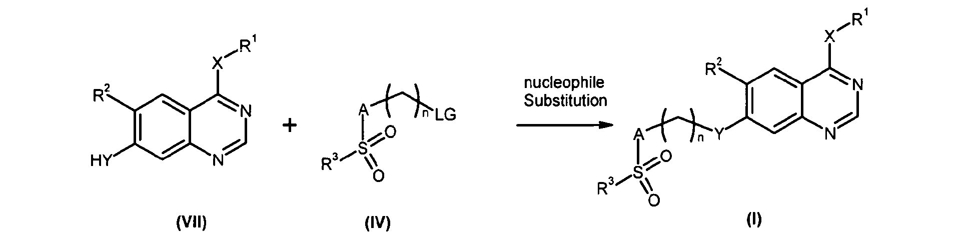 wobei R¹, R², R³ sowie X, Y, A und n die in der allgemeinen Formel (I) gemäß der Ansprüche 1 bis 11 angegebenen Bedeutungen haben.

14. Verfahren zur Herstellung der Verbindungen gemäß einem der Ansprüche 1 bis 11, **gekennzeichnet durch** eine Oxidation der Intermediaten der Formel (VII) zum entsprechen Sulfon, 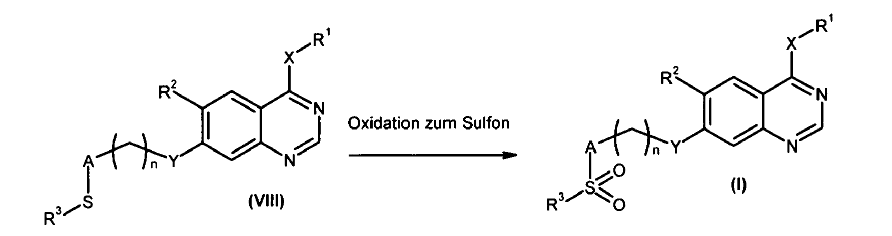 wobei R¹, R², R³ , sowie X, Y, A und n die in der allgemeinen Formel (I) gemäß der Ansprüche 1 bis 11 angegebenen Bedeutungen haben.

15. Intermediate der Formel (II) : 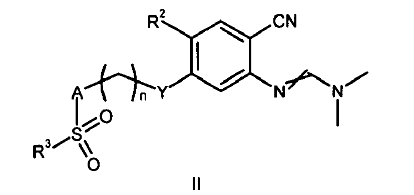 wobei R², R³ sowie Y, A und n die in der allgemeinen Formel (I) gemäß der Ansprüche 1 bis 11 angegebenen Bedeutungen haben.

16. Verbindungen gemäß einem der Ansprüche 1 bis 11 zur Verwendung als Arzneimittel.

17. Verwendung einer Verbindung gemäß einem der Ansprüche 1 bis 11 zur Herstellung eines Medikamentes zur Behandlung von Erkrankungen, die mit entzündlichen, allergischen und / oder proliferativen Prozessen einhergehen.

18. Pharmazeutische Formulierung enthaltend eine Verbindung gemäß einem der Ansprüche 1 bis 11.
